(19) Europäisches Patentamt / European Patent Office / Office européen des brevets

(11) **EP 4 400 059 A1**

(12) **EUROPEAN PATENT APPLICATION**
published in accordance with Art. 153(4) EPC

(43) Date of publication:
**17.07.2024 Bulletin 2024/29**

(21) Application number: **22875872.8**

(22) Date of filing: **15.09.2022**

(51) International Patent Classification (IPC):
**A61B 8/12** (2006.01)

(52) Cooperative Patent Classification (CPC):
**A61B 8/12**

(86) International application number:
**PCT/JP2022/034644**

(87) International publication number:
**WO 2023/054001 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2021 JP 2021160100
29.09.2021 JP 2021160102**

(71) Applicant: **Terumo Kabushiki Kaisha
Tokyo 151-0072 (JP)**

(72) Inventors:
• **SAKAMOTO Yasukazu
Sakai-shi, Osaka 599-8232 (JP)**
• **SHIMIZU Katsuhiko
Ashigarakami-gun, Kanagawa 259-0151 (JP)**
• **ISHIHARA Hiroyuki
Ashigarakami-gun, Kanagawa 259-0151 (JP)**
• **YOSHIZAWA Shunsuke
Ashigarakami-gun, Kanagawa 259-0151 (JP)**

(74) Representative: **Prüfer & Partner mbB
Patentanwälte · Rechtsanwälte
Sohnckestraße 12
81479 München (DE)**

(54) **IMAGE PROCESSING DEVICE, IMAGE PROCESSING SYSTEM, IMAGE DISPLAY METHOD, AND IMAGE PROCESSING PROGRAM**

(57) An image processing device that displays an image representing biological tissue on a display and on the basis of tomographic data acquired from a sensor that moves in the lumen of said biological tissue, indicates the position of the sensor on the same screen as the image, and displays, on the display, a first element that shifts in conjunction with the movement of the sensor, said image processing device comprising: a control unit that, if a user operation for requesting marking of the position of the sensor is received, displays together with the first element on the display, a second element fixed at the same position as the position of the first element when said user operation was performed.

*FIG. 2*

**Description**

Technical Field

The present disclosure relates to an image processing device, an image processing system, an image display method, and an image processing program.

Background Art

Patent Literatures 1 to 3 disclose a technique of generating a three-dimensional image of a cardiac cavity or a blood vessel by using a US image system. "US" is an abbreviation for ultrasound.

Citation List

Patent Literature

Patent Literature 1: US 2010/0215238 A
Patent Literature 2: US 6385332
Patent Literature 3: US 6251072

Summary of Invention

Technical Problem

Treatment using IVUS is widely executed for a cardiac cavity, a cardiac blood vessel, a lower limb artery region, and the like. "IVUS" is an abbreviation for intravascular ultrasound. IVUS is a device or a method for providing a two-dimensional image of a plane perpendicular to a long axis of a catheter.

IVUS is often used for procedures using a catheter separate from an IVUS catheter, such as ablation. For example, in a transatrial septal approach in atrial fibrillation ablation or the like, what is called a Brockenbrough method is used in which a path from the right atrium to the left atrium is formed by puncturing the oval fossa with a septal puncture needle inserted into the right atrium. At the time of puncture, there is a risk of complications such as perforation or cardiac tamponade, and thus it is desirable to sufficiently check the puncture position. In that respect, the IVUS catheter that obtains 360-degree information is excellent in confirming the puncture position in the same plane. However, when IVUS is used, it is difficult to image three-dimensional structure because images are intermittently acquired along the IVUS catheter axis. As a result, there is a possibility that the confirmation of the puncture position in the axial direction becomes insufficient.

An object of the present disclosure is to facilitate confirmation of a position in a biological tissue.

Solution to Problem

An image processing device as one aspect of the present disclosure is an image processing device configured to cause a display to display, based on tomographic data acquired by a sensor moving in a lumen of a biological tissue, an image representing the biological tissue and display a first element on a screen same as the image, the first element representing a position of the sensor and being displaced as the sensor moves, the image processing device including a control unit configured to cause the display to display, upon receiving a user operation of requesting marking of the position of the sensor, a second element together with the first element, the second element being fixed at a position same as a position of the first element at time of the user operation.

As one embodiment, the control unit is configured to set a color of the second element to a color different from a color of the first element.

As one embodiment, the control unit is configured to move the sensor to a position corresponding to a position of the second element upon receiving an operation of requesting movement of the sensor to the position corresponding to the position of the second element.

As one embodiment, the control unit is configured to cause the display to display, upon receiving the user operation again, a third element together with the first element and the second element, the third element being fixed at a position same as a position of the first element at time of the user operation performed again.

As one embodiment, the control unit is configured to set a color of the third element to a color different from the color of the second element.

As one embodiment, the control unit is configured to cause the display to display a fourth element together with the first element, the second element, and the third element, the fourth element being fixed at a position between the second element and the third element.

As one embodiment, the control unit is configured to calculate an intermediate position between the second element and the third element as the position between the second element and the third element.

As one embodiment, the control unit is configured to set a color of the fourth element to a color different from the color of the second element and the color of the third element.

As one embodiment, the control unit is configured to move the sensor to a position corresponding to a position of the fourth element upon receiving an operation of requesting movement of the sensor to the position corresponding to the position of the fourth element.

As one embodiment, the control unit is configured to set a color of a region between a cross section corresponding to the position of the second element and a cross section corresponding to a position of the third element to a color different from a color of an adjacent region in a three-dimensional image that is the image.

As one embodiment, the control unit is configured to combine a graphic element group that is an element group including the first element and the second element and an elongated graphic element representing a movement range of the sensor and to cause the display to display the graphic element group and the elongated graphic element.

As one embodiment, the control unit is configured to cause the display to display the elongated graphic element in a direction in which a long axis direction of the elongated graphic element is parallel to a longitudinal direction of the lumen in the three-dimensional image that is the image.

As one embodiment, the control unit is configured to define the first element and the second element in a three-dimensional image that is the image, the first element being defined as at least a voxel representing an inner surface of the biological tissue or a voxel adjacent to the voxel representing the inner surface and representing the lumen in a first voxel group corresponding to a position of the sensor, the second element being defined as at least a voxel representing the inner surface or a voxel adjacent to the voxel representing the inner surface and representing the lumen in a second voxel group corresponding to a position of the sensor at time of the user operation, and colors the second element distinguishably from the first element.

As one embodiment, the control unit is configured to receive an operation of pressing one or more predetermined keys as the user operation.

An image processing system as one aspect of the present disclosure includes the image processing device, and a probe including the sensor.

As one embodiment, the image processing system further includes the display.

An image display method as one aspect of the present disclosure is an image display method of causing a display to display, based on tomographic data acquired by a sensor moving in a lumen of a biological tissue, an image representing the biological tissue and display a first element on a screen same as the image, the first element representing a position of the sensor and being displaced as the sensor moves, the image display method including: receiving a user operation of requesting marking of the position of the sensor; and causing the display to display a second element together with the first element, the second element being fixed at a position same as a position of the first element at time of the user operation.

An image processing program as one aspect of the present disclosure causes a computer to execute processing, the computer causing a display to display, based on tomographic data acquired by a sensor moving in a lumen of a biological tissue, an image representing the biological tissue and display a first element on a screen same as the image, the first element representing a position of the sensor and being displaced as the sensor moves, the processing including causing the display to display, upon reception of a user operation of requesting marking of the position of the sensor, a second element together with the first element, the second element being fixed at a position same as a position of the first element at time of the user operation.

Advantageous Effects of Invention

According to the present disclosure, it is easy to confirm a position in a biological tissue.

Brief Description of Drawings

Fig. 1 is a perspective view of an image processing system according to embodiments of the present disclosure.
Fig. 2 is a diagram illustrating an example of a screen displayed on a display by an image processing system according to a first embodiment of the present disclosure.

Fig. 3 is a diagram illustrating examples of a two-dimensional image displayed on the display by the image processing system according to the first embodiment of the present disclosure.

Fig. 4 is a diagram illustrating an example of a cutting region formed by the image processing system according to the embodiments of the present disclosure.

Fig. 5 is a block diagram illustrating a configuration of an image processing device according to the embodiments of the present disclosure.

Fig. 6 is a diagram illustrating an example of a screen displayed on the display by an image processing system according to a modification of the first embodiment of the present disclosure.

Fig. 7 is a perspective view of a probe and a drive unit according to the embodiments of the present disclosure.

Fig. 8 is a flowchart illustrating an operation of the image processing system according to the embodiments of the present disclosure.

Fig. 9 is a flowchart illustrating an operation of the image processing system according to the embodiments of the present disclosure.

Fig. 10 is a diagram illustrating a result of binarizing a cross-sectional image of a biological tissue in the embodiments of the present disclosure.

Fig. 11 is a diagram illustrating a result of extracting a point cloud of an inner surface of the biological tissue in the embodiments of the present disclosure.

Fig. 12 is a diagram illustrating a result of calculating centroid positions of a cross section of the biological tissue in the embodiments of the present disclosure.

Fig. 13 is a diagram illustrating a result of calculating centroid positions of a plurality of cross sections of the biological tissue in the embodiments of the present disclosure.

Fig. 14 is a diagram illustrating a result of smoothing the result of Fig. 13.

Fig. 15 is a flowchart illustrating an operation of the image processing system according to the first embodiment of the present disclosure.

Fig. 16 is a diagram illustrating an example of a screen displayed on the display by the image processing system according to a modification of the first embodiment of the present disclosure.

Fig. 17 is a block diagram illustrating a configuration of an image processing device according to a modification of the first embodiment of the present disclosure.

Fig. 18 is a diagram illustrating an example of a screen displayed on the display by an image processing system according to a second embodiment of the present disclosure.

Fig. 19 is a diagram illustrating an example of a screen displayed on the display by the image processing system according to the second embodiment of the present disclosure.

Fig. 20 is a diagram illustrating examples of a two-dimensional image displayed on the display by the image processing system according to the second embodiment of the present disclosure.

Fig. 21 is a flowchart illustrating an operation of the image processing system according to the second embodiment of the present disclosure.


Description of Embodiments


Hereinafter, a first embodiment as one embodiment of the present disclosure will be described with reference to the drawings.

In the drawings, the same or corresponding portions are denoted by the same reference numerals. In the description of the present embodiment, description of the same or corresponding parts will be omitted or simplified as appropriate.

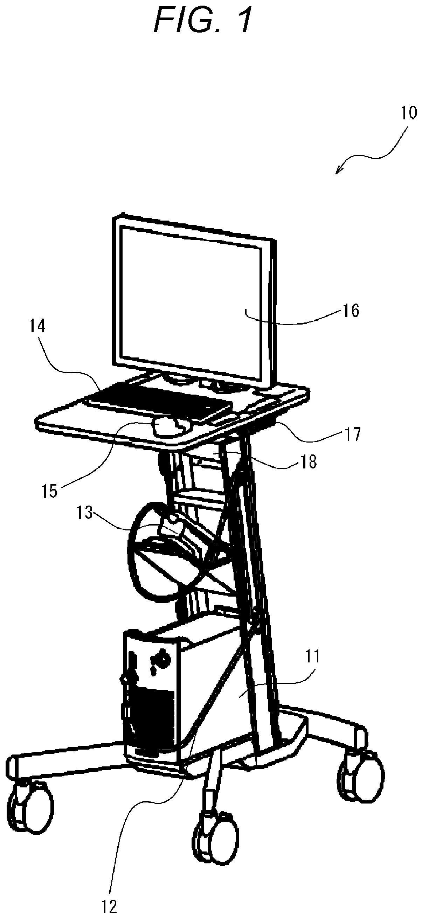

An outline of the present embodiment will be described with reference to Figs. 1 to 5.

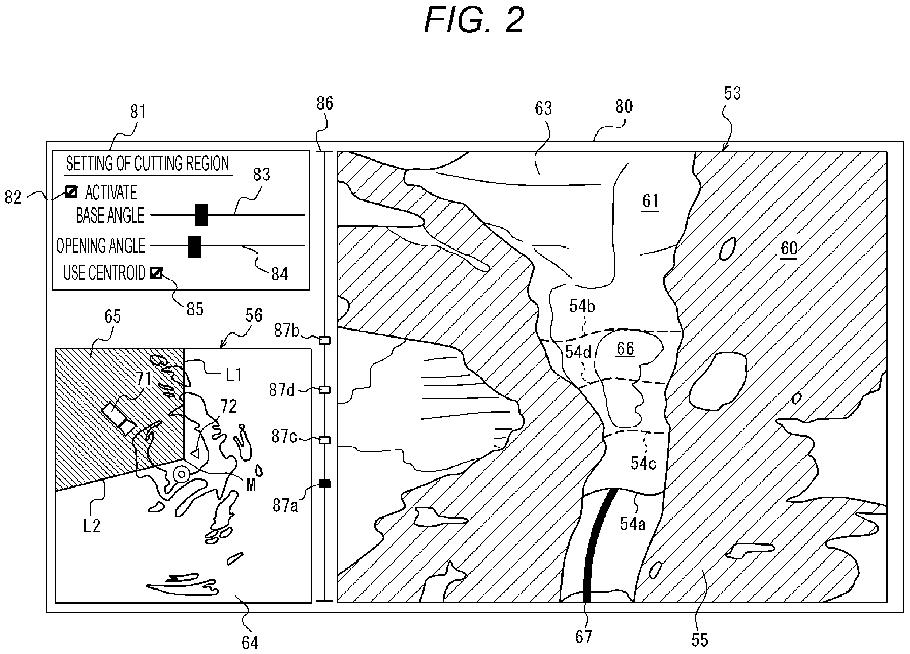

An image processing device 11 according to the present embodiment is a computer that causes a display 16 to display, based on tomographic data 51 acquired by a sensor moving in a lumen 63 of a biological tissue 60, an image representing the biological tissue 60 and display a first element on a screen 80 same as the image, the first element representing a position of the sensor and being displaced as the sensor moves. In the present embodiment, the image representing the biological tissue 60 is a three-dimensional image 53 as illustrated on the right part of Fig. 2, but may be a two-dimensional image 56 such as a cross-sectional image as illustrated on the left part of Fig. 2. In the present embodiment, the first element is a graphic element such as a slider knob as illustrated in Fig. 2 as a first graphic element 87a, but may be voxels colored in a first color such as white in a first voxel group 54a corresponding to the position of the sensor. Upon receiving a marking operation that is a user operation of requesting marking of the position of the sensor, the image processing device 11 causes the display 16 to display a second element together with the first element, the second element being fixed at the same position as the position of the first element at the time of the marking operation. In the present embodiment, the second element is a graphic element such as a rectangular mark as illustrated in Fig. 2 as a second graphic element 87b, but may be voxels colored in a second color such as green in a second voxel group 54b corresponding to the position of the sensor at the time of the marking operation.

When receiving a marking operation again, the image processing device 11 causes the display 16 to display a third element together with the first element and the second element, the third element being fixed at the same position as the position of the first element at the time of the marking operation performed again. In the present embodiment, the third element is a graphic element such as a rectangular mark as illustrated in Fig. 2 as a third graphic element 87c, but may be voxels colored in a third color such as red in a third voxel group 54c corresponding to the position of the sensor at the time of the marking operation performed again.

The image processing device 11 causes the display 16 to display a fourth element together with the first element, the second element, and the third element, the fourth element being fixed at a position between the second element and the third element. In the present embodiment, the fourth element is a graphic element such as a rectangular mark as illustrated in Fig. 2 as a fourth graphic element 87d, but may be voxels colored in a fourth color such as yellow in a fourth voxel group 54d existing between the second voxel group 54b and the third voxel group 54c, specifically, existing in the middle thereof.

According to the present embodiment, it is easy to confirm a position in the biological tissue 60. For example, when a procedure such as ablation using IVUS is performed, an axis synchronized with the linear scaler of the pullback unit of IVUS is displayed as a fifth graphic element 86 on the screen 80, specifically, next to the two-dimensional image 56. The current position of the ultrasound element, which is an IVUS sensor, is always displayed on the axis as the first graphic element 87a. A user, such as a physician operating a catheter while performing a procedure or a clinical technician operating an IVUS system while watching the display 16, can mark the current position of the ultrasound element on the axis by performing a marking operation that is an operation of pressing one or more predetermined keys, such as simultaneously pressing the Ctrl key and "B". For example, when the septal paracentesis is performed, the position of the upper end of an oval fossa 66 can be marked on the axis as the second graphic element 87b by performing a marking operation when the upper end of the oval fossa 66 is detected by the IVUS. By performing the marking operation again when IVUS is detecting the lower end of the oval fossa 66, the position of the lower end of the oval fossa 66 can be further marked on the axis as the third graphic element 87c. If the intermediate position between the upper end and the lower end of the oval fossa 66 is automatically calculated, the intermediate position between the upper end and the lower end of the oval fossa 66 can be further marked on the axis as the fourth graphic element 87d. Therefore, the center position of the oval fossa 66 can be easily confirmed as the puncture position. As a result, the risk of complications can be reduced.

In the present embodiment, the axis is directly displayed as the fifth graphic element 86, but the three-dimensional image 53 can also be considered to correspond to the axis. That is, in the three-dimensional image 53, since the first voxel group 54a is a voxel group on the same plane as the current position of the ultrasound element, it may be considered that the voxels colored in the first color in the first voxel group 54a are always displayed on the axis as a colored line indicating the current position of the ultrasound element. Assuming that the second voxel group 54b is a voxel group that is on the same plane as the ultrasound element when the upper end of the oval fossa 66 is detected by IVUS and a marking operation is performed, it may be considered that the voxels colored in the second color in the second voxel group 54b are displayed on the axis as a colored line indicating the position of the upper end of the oval fossa 66. Assuming that the third voxel group 54c is a voxel group that is on the same plane as the ultrasound element when the lower end of the oval fossa 66 is detected by IVUS and the marking operation is performed again, it may be considered that the voxels colored in the third color in the third voxel group 54c are displayed on the axis as a colored line indicating the position of the lower end of the oval fossa 66. Since the fourth voxel group 54d is a voxel group existing in the middle of the second voxel group 54b and the third voxel group 54c, it may be considered that the voxels colored in the fourth color in the fourth voxel group 54d are displayed on the axis as a colored line indicating the intermediate position between the upper end and the lower end of the oval fossa 66.

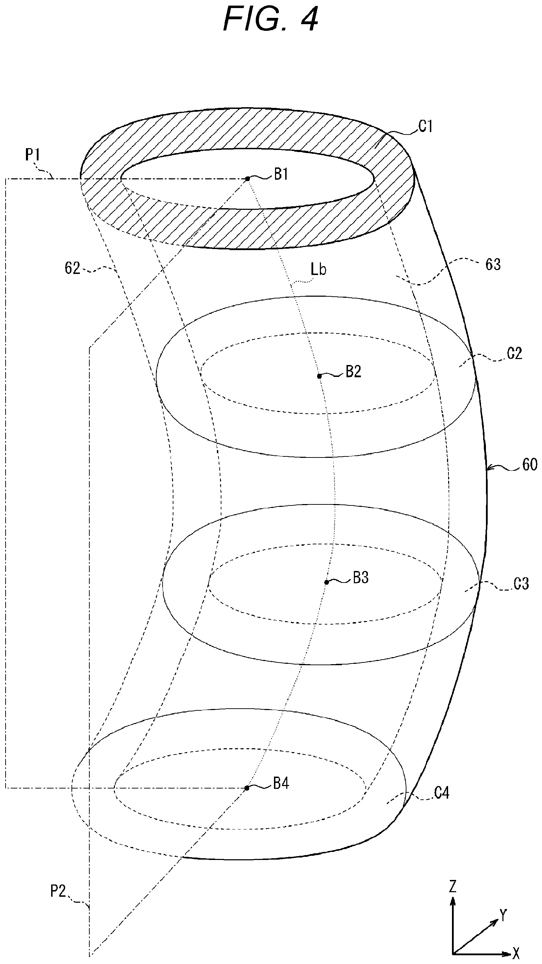

The image processing device 11 causes the display 16 to display the three-dimensional data 52 representing the biological tissue 60 as a three-dimensional image 53. As illustrated in Fig. 4, the image processing device 11 forms, in the three-dimensional data 52, a cutting region 62 exposing the lumen 63 of the biological tissue 60 on the three-dimensional image 53. As illustrated in Fig. 2, the image processing device 11 causes the display 16 to display the two-dimensional image 56 representing a cross section 64 of the biological tissue 60 and a region 65 corresponding to the cutting region 62 in the cross section 64 together with the three-dimensional image 53.

According to the present embodiment, how a part of the structure of the biological tissue 60 is cut can be indicated. Therefore, the user can grasp from the two-dimensional image 56 what type of structure the portion of the biological tissue 60 that is not cut or displayed in the three-dimensional image 53 is. For example, in a case where the user is an operator, an operation on the inside of the biological tissue 60 can be easily performed.

The image processing device 11 causes the display 16 to display a three-dimensional object representing an elongated medical instrument 67 inserted into the lumen 63 so as to be included in the three-dimensional image 53. In the present embodiment, the elongated medical instrument 67 is a catheter in which a puncture needle is attached to the distal end, but may be another type of medical instrument such as a guide wire as long as it is elongated.

The image processing device 11 disposes a mark 72 representing the distal end of the elongated medical instrument 67 on the two-dimensional image 56. The mark 72 may be a mark of any color and shape, and in the example of Fig. 2, it is a solid yellow triangle. The mark 72 is directed in an ultrasound wave emission direction from a centroid of the cross section 64 of the biological tissue 60 into which the catheter as the elongated medical instrument 67 is inserted. Therefore, the user can easily recognize that the mark 72 is the puncture needle.

The image processing device 11 generates and updates the three-dimensional data 52 based on the tomographic data 51. As illustrated in Fig. 2, in the three-dimensional image 53, the image processing device 11 defines, as the first element, at least a voxel representing an inner surface 61 of the biological tissue 60 or a voxel adjacent to the voxel representing the inner surface 61 and representing the lumen 63 in the first voxel group 54a corresponding to the position of the sensor, and colors the first element distinguishably from other voxel groups 55. After the marking operation is performed, the image processing device 11 defines, as the second element, at least a voxel representing the inner surface 61 or a voxel adjacent to the voxel representing the inner surface 61 and representing the lumen 63 in the second voxel group 54b corresponding to the position of the sensor that is the first voxel group 54a at the time of the marking operation, and colors the second element distinguishably from other voxel groups including the first element. After the marking operation is performed again, the image processing device 11 defines, as the third element, at least a voxel representing the inner surface 61 or a voxel adjacent to the voxel representing the inner surface 61 and representing the lumen 63 in the third voxel group 54c corresponding to the position of the sensor at the time of the marking operation performed again, and colors the third element distinguishably from other voxel groups including the first element and the second element. In the present embodiment, the image processing device 11 defines, as the fourth element, at least a voxel representing the inner surface 61 or a voxel adjacent to the voxel representing the inner surface 61 and representing the lumen 63 in the fourth voxel group 54d existing in the middle of the second voxel group 54b and the third voxel group 54c, and colors the fourth element distinguishably from other voxel groups including the first element, the second element, and the third element.

According to the present embodiment, which portion in the three-dimensional image 53 corresponds to the cross section 64 of the biological tissue 60 indicated by the tomographic data 51 newly acquired by the sensor can be indicated. Therefore, it is easy for the user observing the lumen 63 of the biological tissue 60 using the three-dimensional image 53 to grasp which portion in the three-dimensional image 53 corresponds to information currently obtained by the sensor, that is, the latest information. Also for the first marking point such as the upper end of the oval fossa 66, it is possible to indicate which part in the three-dimensional image 53 corresponds to the point. Also for the second marking point such as the lower end of the oval fossa 66, it is possible to indicate which part in the three-dimensional image 53 corresponds to the point. Also for a particular position between the two marking points, such as the intermediate position between the upper end and the lower end of the oval fossa 66, it is possible to indicate which part in the three-dimensional image 53 corresponds to the position. Therefore, it is easy to confirm the position in the biological tissue 60 in the axial direction, such as the puncture position in the axial direction.

In a modification of the present embodiment, not only the first voxel group 54a, but also at least a voxel representing the inner surface 61 or a voxel adjacent to the voxel representing the inner surface 61 and representing the lumen 63 in a voxel group corresponding to a cross section adjacent to the cross section 64 to which the first voxel group 54a corresponds may be colored distinguishably from the voxel groups corresponding to other cross sections of the biological tissue 60. According to this modification, the width in the movement direction of the sensor of the voxel group colored distinguishably from the voxel groups corresponding to other cross sections is widened, and the user can easily recognize the voxel group in the three-dimensional image 53.

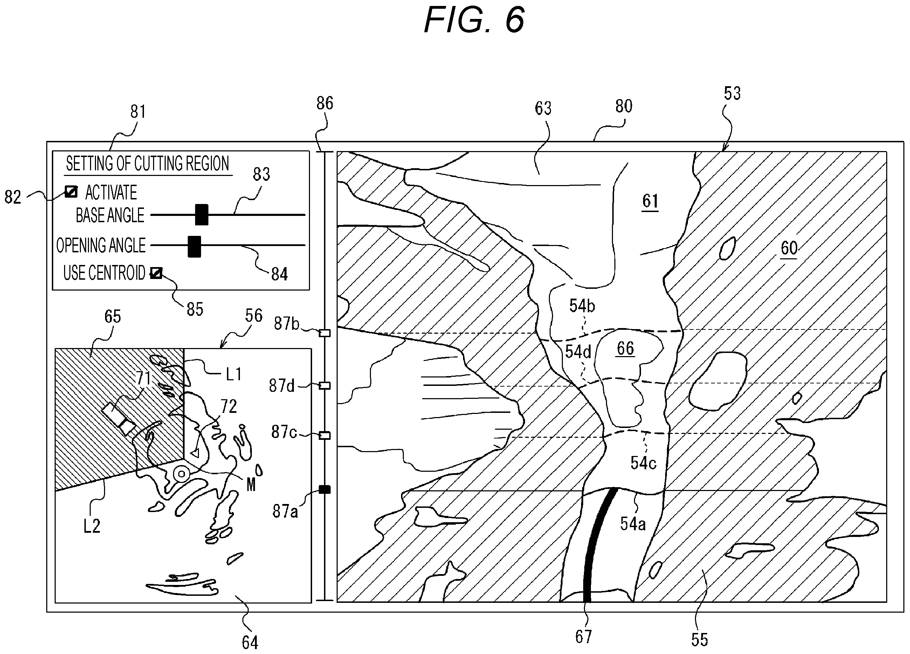

In a modification of the present embodiment, as illustrated in Fig. 6, all voxels representing the biological tissue 60 in the first voxel group 54a may be defined as the first element and colored distinguishably from the other voxel groups 55. After the marking operation is performed, all the voxels representing the biological tissue 60 in the second voxel group 54b may be defined as the second element and colored distinguishably from other voxel groups including the first voxel group 54a. After the marking operation is performed again, all the voxels representing the biological tissue 60 in the third voxel group 54c may be defined as the third element and colored distinguishably from other voxel groups including the first voxel group 54a and the second voxel group 54b. All the voxels representing the biological tissue 60 in the fourth voxel group 54d may be defined as the fourth element and colored distinguishably from other voxel groups including the first voxel group 54a, the second voxel group 54b, and the third voxel group 54c. According to this modification, since the first voxel group 54a is colored distinguishably from the other voxel groups 55 also on the cutting plane of the biological tissue 60 formed for observing the lumen 63 of the biological tissue 60, it is easier for the user to grasp which portion in the three-dimensional image 53 corresponds to the latest information. On the cutting plane, not only the first voxel group 54a but also the second voxel group 54b, the third voxel group 54c, and the fourth voxel group 54d are colored distinguishably from other voxel groups, so that the position in the biological tissue 60 in the axial direction can be more easily confirmed.

In the present embodiment, the image processing device 11 causes the display 16 to display the two-dimensional image 56 representing the cross section 64 together with the three-dimensional image 53 in which at least a voxel representing the inner surface 61 of the biological tissue 60 or a voxel adjacent to the voxel representing the inner surface 61 and representing the lumen 63 in the first voxel group 54a corresponding to the cross section 64 is colored distinguishably from the voxel groups corresponding to other cross sections. Therefore, the relation between the two-dimensional image 56 and the three-dimensional image 53 can be indicated.

The biological tissue 60 includes, for example, an organ such as a blood vessel or a heart. The biological tissue 60 is not limited to only an anatomically single organ or a part thereof, but also includes a tissue including a lumen across a plurality of organs. An example of such a tissue is, specifically, a part of a vascular tissue extending from an upper part of an inferior vena cava to a lower part of a superior vena cava through a right atrium.

In Fig. 2, an operation panel 81, the two-dimensional image 56, the three-dimensional image 53, the first graphic element 87a, the second graphic element 87b, the third graphic element 87c, the fourth graphic element 87d, and the fifth graphic element 86 are displayed on the screen 80.

The operation panel 81 is a GUI component for setting the cutting region 62. "GUI" is an abbreviation for graphical user interface. The operation panel 81 includes a check box 82 for selecting whether to activate settings of the cutting region 62, a slider 83 for setting a base angle, a slider 84 for setting an opening angle, and a check box 85 for selecting whether to use a centroid.

The base angle is a rotation angle of one straight line L1 of two straight lines L1 and L2 extending from one point M in the cross-sectional image representing the cross section 64 of the biological tissue 60. Therefore, setting the base angle corresponds to setting the direction of the straight line L1. The opening angle is an angle between the two straight lines L1 and L2. Therefore, setting the opening angle corresponds to setting the angle formed by the two straight lines L1 and L2. The point M is the centroid of the cross section 64. In a case where non-use of a centroid is selected, the point M may be set to a point other than the centroid on the cross section 64.

The two-dimensional image 56 is an image obtained by processing a cross-sectional image. In the two-dimensional image 56, the color of the region 65 corresponding to the cutting region 62 is changed in order to clearly indicate which portion of the cross section 64 is cut.

In the present embodiment, the viewpoint for displaying the three-dimensional image 53 on the screen 80 is adjusted according to the position of the cutting region 62. The viewpoint is a position of a virtual camera 71 disposed in a three-dimensional space. In the two-dimensional image 56, the position of the camera 71 with respect to the cross section 64 is displayed.

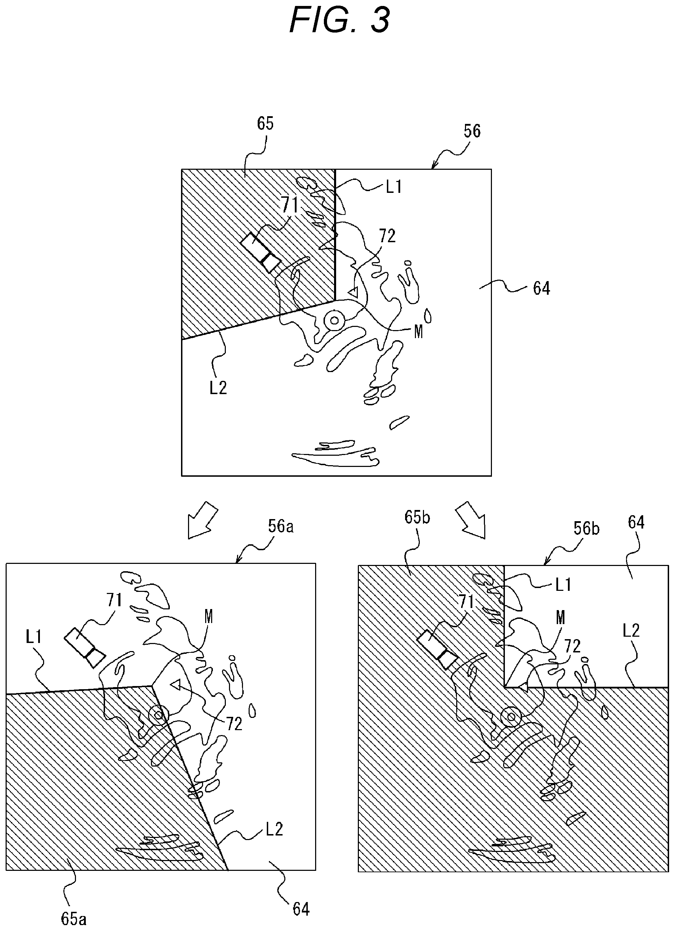

In the present embodiment, the cutting region 62 can be determined using the two-dimensional image 56. Specifically, as illustrated in Fig. 3, the position or size of the cutting region 62 can be set by adjusting the base angle or the opening angle to set the position or size of the region 65 obtained by being divided by the two straight lines L1 and L2 in the two-dimensional image 56. For example, in a case where the base angle is changed such that the straight line L1 rotates counterclockwise by about 90 degrees, a region 65a moved according to the change of the base angle is obtained in a two-dimensional image 56a. Then, the position of the cutting region 62 is adjusted according to the position of the region 65a. Alternatively, in a case where the opening angle is changed such that the angle between the two straight lines L1 and L2 is increased, a region 65b enlarged according to the change in the opening angle is obtained in a two-dimensional image 56b. Then, the size of the cutting region 62 is adjusted according to the size of the region 65b. Both the position and the size of the cutting region 62 can be set by adjusting both the base angle and the opening angle to set both the position and the size of the region 65 in the two-dimensional image 56. The position of the camera 71 may be appropriately adjusted according to the position or size of the cutting region 62.

In the present embodiment, an image corresponding to the current position of the sensor, that is, the latest image is always displayed as the two-dimensional image 56.

In a modification of the present embodiment, the base angle may be set by dragging the straight line L1 or by inputting a numerical value instead of being set by operating the slider 83. Similarly, the opening angle may be set by dragging the straight line L2 or by inputting a numerical value instead of being set by operating the slider 84.

In the three-dimensional image 53, the cutting region 62 determined using the two-dimensional image 56 is hidden or transparent. In the three-dimensional image 53, to express the position at which the sensor is currently present in the longitudinal direction of the lumen 63 and update is currently performed in real time, the color of the first voxel group 54a corresponding to the current position of the sensor is changed.

In the present embodiment, as illustrated in Fig. 2, voxels representing the inner surface 61 of the biological tissue 60 in the first voxel group 54a are set to a color different from that of the other voxel groups 55, so that the voxels are colored distinguishably from the other voxel groups 55. However, in a modification of the present embodiment, as illustrated in Fig. 6, all voxels representing the biological tissue 60 in the first voxel group 54a may be set to a different color. In another modification, instead of setting the first voxel group 54a and the other voxel groups 55 to different colors, the first voxel group 54a may be colored distinguishably from the other voxel groups 55 by adjusting the contrast between the first voxel group 54a and the other voxel groups 55. The second voxel group 54b, the third voxel group 54c, and the fourth voxel group 54d are also similar to the first voxel group 54a.

The first graphic element 87a is a graphic element representing the position of the sensor. The fifth graphic element 86 is an elongated graphic element representing a movement range of the sensor. In the present embodiment, a combination of the first graphic element 87a and the fifth graphic element 86 is configured as a slider. The first graphic element 87a and the fifth graphic element 86 may be displayed at any position, but are displayed between the two-dimensional image 56 and the three-dimensional image 53 in the present embodiment.

The second graphic element 87b is a graphic element fixed at the same position as the position of the first graphic element 87a at the time of the marking operation. The third graphic element 87c is a graphic element fixed at the same position as the position of the first graphic element 87a at the time of the marking operation performed again. The fourth graphic element 87d is a graphic element fixed at the position between the second graphic element 87b and the third graphic element 87c.

In the present embodiment, when the pullback operation is performed, the relative position of the first graphic element 87a is changed in accordance with the movement of the sensor. For example, assuming that no pullback operation is currently performed, as illustrated in Fig. 2, voxels representing the inner surface 61 of the biological tissue 60 in the first voxel group 54a corresponding to the current position of the sensor are colored in the first color such as white. The first graphic element 87a is also displayed in the first color. The first graphic element 87a represents the current position of the sensor by being located at the same height as the first voxel group 54a.

If the sensor detects the upper end of the oval fossa 66 and a marking operation such as simultaneously pressing the Ctrl key and "B" is performed as the user operation, the second graphic element 87b is newly displayed. Voxels representing the inner surface 61 of the biological tissue 60 in the second voxel group 54b corresponding to the position of the upper end of the oval fossa 66 are colored in the second color such as green. The second graphic element 87b is also displayed in the second color. The second graphic element 87b represents the position of the upper end of the oval fossa 66 by being located at the same height as the second voxel group 54b.

If the sensor detects the lower end of the oval fossa 66 and the marking operation such as simultaneously pressing the Ctrl key and "B" is performed again as the user operation, the third graphic element 87c is newly displayed. Voxels representing the inner surface 61 of the biological tissue 60 in the third voxel group 54c corresponding to the position of the lower end of the oval fossa 66 are colored in the third color such as red. The third graphic element 87c is also displayed in the third color. The third graphic element 87c represents the position of the lower end of the oval fossa 66 by being located at the same height as the third voxel group 54c.

When the calculation of the midpoint between the position of the second graphic element 87b and the position of the third graphic element 87c is completed, the fourth graphic element 87d is newly displayed. Voxels representing the inner surface 61 of the biological tissue 60 in the fourth voxel group 54d corresponding to the intermediate position between the upper end and the lower end of the oval fossa 66 are colored in the fourth color such as yellow. The fourth graphic element 87d is also displayed in the fourth color. The fourth graphic element 87d represents the intermediate position between the upper end and the lower end of the oval fossa 66, that is, a desired puncture position, by being located at the same height as the fourth voxel group 54d.

In Fig. 4, an X direction and a Y direction orthogonal to the X direction correspond to the lateral directions of the lumen 63 of the biological tissue 60. A Z direction orthogonal to the X direction and the Y direction corresponds to the longitudinal direction of the lumen 63 of the biological tissue 60.

In the example of Fig. 4, it is assumed that the check box 85 of the operation panel 81 is in a checked state, that is, use of a centroid is selected. The image processing device 11 calculates the positions of centroids B1, B2, B3, and B4 of respective cross sections C1, C2, C3, and C4 of the biological tissue 60 using the three-dimensional data 52. The image processing device 11 sets two planes intersecting at a single line Lb passing through the positions of the centroids B1, B2, B3, and B4 and including, respectively, the two straight lines L1 and L2 as cutting planes P1 and P2. For example, assuming that the point M illustrated in Fig. 2 is the point B3, the straight line L1 is an intersection line between the cross section C3 and the cutting plane P1, and the straight line L2 is an intersection line between the cross section C3 and the cutting plane P2. The image processing device 11 forms, in the three-dimensional data 52, a region interposed between the cutting planes P1 and P2 and exposing the lumen 63 of the biological tissue 60 in the three-dimensional image 53, as the cutting region 62.

In the case of a three-dimensional model of the biological tissue 60 bent as illustrated in Fig. 4, when the three-dimensional model is cut along a single plane to display the lumen 63, there is a case in which an inside of the biological tissue 60 cannot be correctly displayed. In the present embodiment, as illustrated in Fig. 4, by continuously capturing centroids of cross sections of the biological tissue 60, the three-dimensional model can be cut such that the inside of the biological tissue 60 can be reliably displayed.

In Fig. 4, for convenience, the four cross sections C1, C2, C3, and C4 are illustrated as a plurality of lateral cross sections of the lumen 63 of the biological tissue 60, but the number of cross sections serving as calculation targets of the centroid positions is not limited to four, and is preferably the same number as that of cross-sectional images acquired by IVUS.

In another example different from Fig. 4, it is assumed that the check box 85 of the operation panel 81 is not in a checked state, that is, non-use of a centroid is selected. In such an example, the image processing device 11 sets two planes intersecting at any one line passing through the point M, such as a straight line extending in the Z direction through the point M, and including, respectively, the two straight lines L1 and L2, as the cutting planes P1 and P2.

A configuration of an image processing system 10 according to the present embodiment will be described with reference to Fig. 1.

The image processing system 10 includes the image processing device 11, a cable 12, a drive unit 13, a keyboard 14, a mouse 15, and the display 16.

The image processing device 11 is a dedicated computer specialized for image diagnosis in the present embodiment, but may be a general-purpose computer such as a PC. "PC" is an abbreviation for personal computer.

The cable 12 is used to connect the image processing device 11 and the drive unit 13.

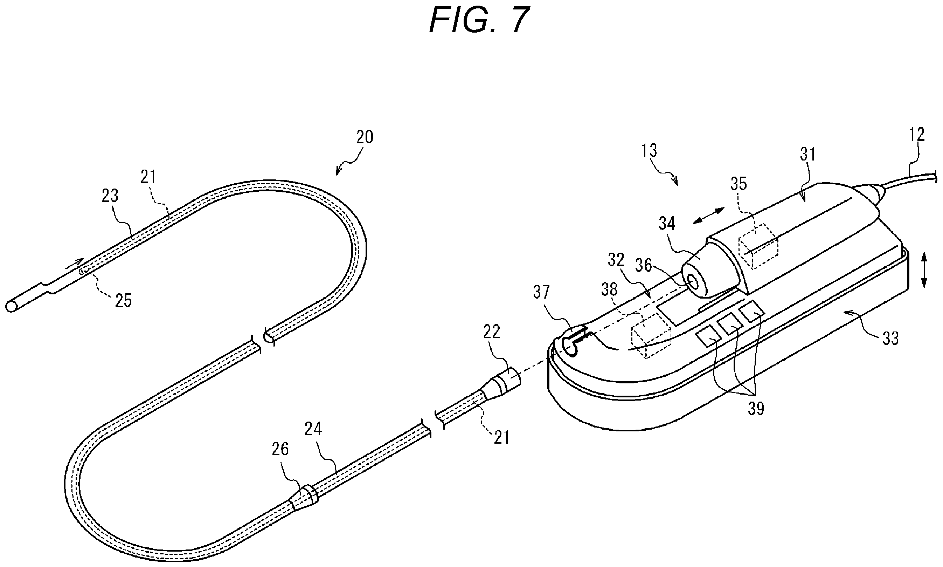

The drive unit 13 is a device that is used by being connected to a probe 20 illustrated in Fig. 7 and drives the probe 20. The drive unit 13 is also referred to as an MDU. "MDU" is an abbreviation for motor drive unit. The probe 20 is applied to IVUS. The probe 20 is also referred to as an IVUS catheter or an image diagnostic catheter.

The keyboard 14, the mouse 15, and the display 16 are connected to the image processing device 11 via a certain cable or wirelessly. The display 16 is, for example, an LCD, an organic EL display, or an HMD. "LCD" is an abbreviation for liquid crystal display. "EL" is an abbreviation for electro luminescence. "HMD" is an abbreviation for head-mounted display.

The image processing system 10 optionally further includes a connection terminal 17 and a cart unit 18.

The connection terminal 17 is used to connect the image processing device 11 and an external device. The connection terminal 17 is, for example, a USB terminal. "USB" is an abbreviation for universal serial bus. The external device is, for example, a recording medium such as a magnetic disc drive, a magneto-optical disc drive, or an optical disc drive.

The cart unit 18 is a cart equipped with casters for movement. The image processing device 11, the cable 12, and the drive unit 13 are disposed on a cart body of the cart unit 18. The keyboard 14, the mouse 15, and the display 16 are disposed on an uppermost table of the cart unit 18.

Configurations of the probe 20 and the drive unit 13 according to the present embodiment will be described with reference to Fig. 7.

The probe 20 includes a drive shaft 21, a hub 22, a sheath 23, an outer tube 24, an ultrasound transducer 25, and a relay connector 26.

The drive shaft 21 passes through the sheath 23 to be inserted into a lumen of a living body and the outer tube 24 connected to a proximal end of the sheath 23, and extends to an inside of the hub 22 disposed at a proximal end of the probe 20. The drive shaft 21 is provided with the ultrasound transducer 25, which transmits and receives signals, at a distal end of the drive shaft 21, and is rotatably disposed in the sheath 23 and the outer tube 24. The relay connector 26 connects the sheath 23 and the outer tube 24.

The hub 22, the drive shaft 21, and the ultrasound transducer 25 are connected to each other to integrally move forward and backward in an axial direction. Therefore, for example, when the hub 22 is pressed toward the distal side, the drive shaft 21 and the ultrasound transducer 25 move inside the sheath 23 toward the distal side. For example, when the hub 22 is pulled toward the proximal side, the drive shaft 21 and the ultrasound transducer 25 move inside the sheath 23 toward the proximal side as indicated by an arrow.

The drive unit 13 includes a scanner unit 31, a slide unit 32, and a bottom cover 33.

The scanner unit 31 is also referred to as a pullback unit. The scanner unit 31 is connected to the image processing device 11 via the cable 12. The scanner unit 31 includes a probe connection portion 34 connected to the probe 20, and a scanner motor 35 which is a drive source for rotating the drive shaft 21.

The probe connection portion 34 is freely detachably connected to the probe 20 through an insertion port 36 of the hub 22 disposed at the proximal end of the probe 20. Inside the hub 22, the proximal end of the drive shaft 21 is rotatably supported, and the rotational force of the scanner motor 35 is transmitted to the drive shaft 21. A signal is transmitted and received between the drive shaft 21 and the image processing device 11 via the cable 12. In the image processing device 11, generation of a tomographic image of a body lumen and image processing are executed based on the signal transmitted from the drive shaft 21.

The slide unit 32 is mounted with the scanner unit 31 that moves forward and backward, and is mechanically and electrically connected to the scanner unit 31. The slide unit 32 includes a probe clamp unit 37, a slide motor 38, and a switch group 39.

The probe clamp unit 37 is disposed coaxially with the probe connection portion 34 and distal of the probe connection portion 34, and supports the probe 20 to be connected to the probe connection portion 34.

The slide motor 38 is a drive source that generates a driving force in the axial direction. The scanner unit 31 moves forward and backward when driven by the slide motor 38, and the drive shaft 21 moves forward and backward in the axial direction accordingly. The slide motor 38 is, for example, a servo motor.

The switch group 39 includes, for example, a forward switch and a pullback switch that are pressed when the scanner unit 31 is to be moved forward or backward, and a scan switch that is pressed when image drawing is to be started or ended. Various switches are included in the switch group 39 as necessary without being limited to the example here.

When the forward switch is pressed, the slide motor 38 rotates forward, and the scanner unit 31 moves forward. Meanwhile, when the pullback switch is pressed, the slide motor 38 rotates backward, and the scanner unit 31 moves backward.

When the scan switch is pressed, the image drawing is started, the scanner motor 35 is driven, and the slide motor 38 is driven to move the scanner unit 31 backward. The user such as the operator connects the probe 20 to the scanner unit 31 in advance, such that the drive shaft 21 rotates and moves toward the proximal side in the axial direction upon the start of the image drawing. When the scan switch is pressed again, the scanner motor 35 and the slide motor 38 are stopped, and the image drawing is ended.

The bottom cover 33 covers the bottom and the entire circumference of a side surface on a bottom side of the slide unit 32, and can be attached to and removed from the bottom of the slide unit 32.

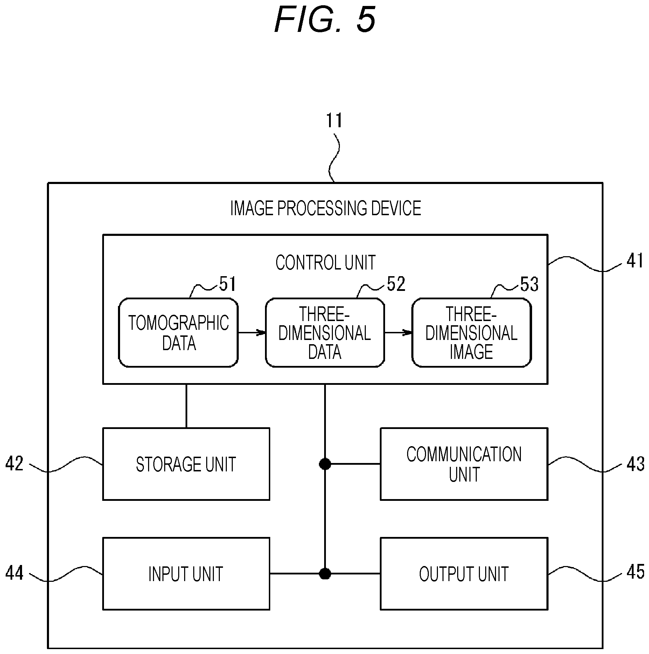

A configuration of the image processing device 11 will be described with reference to Fig. 5.

The image processing device 11 includes a control unit 41, a storage unit 42, a communication unit 43, an input unit 44, and an output unit 45.

The control unit 41 includes at least one processor, at least one programmable circuit, at least one dedicated circuit, or any combination thereof. The processor is a general-purpose processor such as a CPU or GPU, or a dedicated processor specialized for specific processing. "CPU" is an abbreviation for central processing unit. "GPU" is an abbreviation for graphics processing unit. The programmable circuit is, for example, an FPGA. "FPGA" is an abbreviation for field-programmable gate array. The dedicated circuit is, for example, an ASIC. "ASIC" is an abbreviation for application specific integrated circuit. The control unit 41 executes processing related to an operation of the image processing device 11 while controlling each unit of the image processing system 10 including the image processing device 11.

The storage unit 42 includes at least one semiconductor memory, at least one magnetic memory, at least one optical memory, or any combination thereof. The semiconductor memory is, for example, a RAM or a ROM. "RAM" is an abbreviation for random access memory. "ROM" is an abbreviation for read only memory. The RAM is, for example, an SRAM or a DRAM. "SRAM" is an abbreviation for static random access memory. "DRAM" is an abbreviation for dynamic random access memory. The ROM is, for example, an EEPROM. "EEPROM" is an abbreviation for electrically erasable programmable read only memory. The storage unit 42 functions as, for example, a main storage device, an auxiliary storage device, or a cache memory. The storage unit 42 stores data used for the operation of the image processing device 11, such as the tomographic data 51, and data obtained by the operation of the image processing device 11, such as the three-dimensional data 52 and the three-dimensional image 53.

The communication unit 43 includes at least one communication interface. The communication interface is, for example, a wired LAN interface, a wireless LAN interface, or an image diagnostic interface for receiving IVUS signals and executing A/D conversion for the IVUS signals. "LAN" is an abbreviation for local area network. "A/D" is an abbreviation for analog to digital. The communication unit 43 receives data used for the operation of the image processing device 11 and transmits data obtained by the operation of the image processing device 11. In the present embodiment, the drive unit 13 is connected to the image diagnostic interface included in the communication unit 43.

The input unit 44 includes at least one input interface. The input interface is, for example, a USB interface, an HDMI (registered trademark) interface, or an interface compatible with a short-range wireless communication standard such as Bluetooth (registered trademark). "HDMI (registered trademark)" is an abbreviation for high-definition multimedia interface. The input unit 44 receives an operation by the user such as an operation of inputting data used for the operation of the image processing device 11. In the present embodiment, the keyboard 14 and the mouse 15 are connected to the USB interface or the interface compatible with short-range wireless communication included in the input unit 44. When a touch screen is disposed integrally with the display 16, the display 16 may be connected to the USB interface or the HDMI (registered trademark) interface included in the input unit 44.

The output unit 45 includes at least one output interface. The output interface is, for example, a USB interface, an HDMI (registered trademark) interface, or an interface compatible with a short-range wireless communication standard such as Bluetooth (registered trademark). The output unit 45 outputs data obtained by the operation of the image processing device 11. In the present embodiment, the display 16 is connected to the USB interface or the HDMI (registered trademark) interface included in the output unit 45.

A function of the image processing device 11 is implemented by executing an image processing program according to the present embodiment by the processor corresponding to the control unit 41. That is, the function of the image processing device 11 is implemented by software. The image processing program causes a computer to function as the image processing device 11 by causing the computer to execute the operation of the image processing device 11. That is, the computer functions as the image processing device 11 by executing the operation of the image processing device 11 according to the image processing program.

The program may be stored in a non-transitory computer-readable medium in advance. The non-transitory computer-readable medium is, for example, a flash memory, a magnetic recording device, an optical disc, a magneto-optical recording medium, or a ROM. Distribution of the program is executed by, for example, selling, transferring, or lending a portable medium such as an SD card, a DVD, or a CD-ROM storing the program. "SD" is an abbreviation for secure digital. "DVD" is an abbreviation for digital versatile disc. "CD-ROM" is an abbreviation for compact disc read only memory. The program may be distributed by being stored in a storage of a server in advance and transferred from the server to another computer. The program may be provided as a program product.

For example, the computer temporarily stores, in the main storage device, the program stored in the portable medium or the program transferred from the server. Then, the computer reads, by the processor, the program stored in the main storage device, and executes, by the processor, processing according to the read program. The computer may read the program directly from the portable medium and execute the processing according to the program. Each time the program is transferred from the server to the computer, the computer may sequentially execute processing according to the received program. The processing may be executed by what is called an ASP type service in which the function is implemented only by execution instruction and result acquisition without transferring the program from the server to the computer. "ASP" is an abbreviation for application service provider. The program includes information provided for processing by an electronic computer and conforming to the program. For example, data that is not a direct command to the computer but has a property that defines the processing of the computer corresponds to the "information conforming to the program".

The functions of the image processing device 11 may be partially or entirely implemented by the programmable circuit or the dedicated circuit corresponding to the control unit 41. That is, the functions of the image processing device 11 may be partially or entirely implemented by hardware.

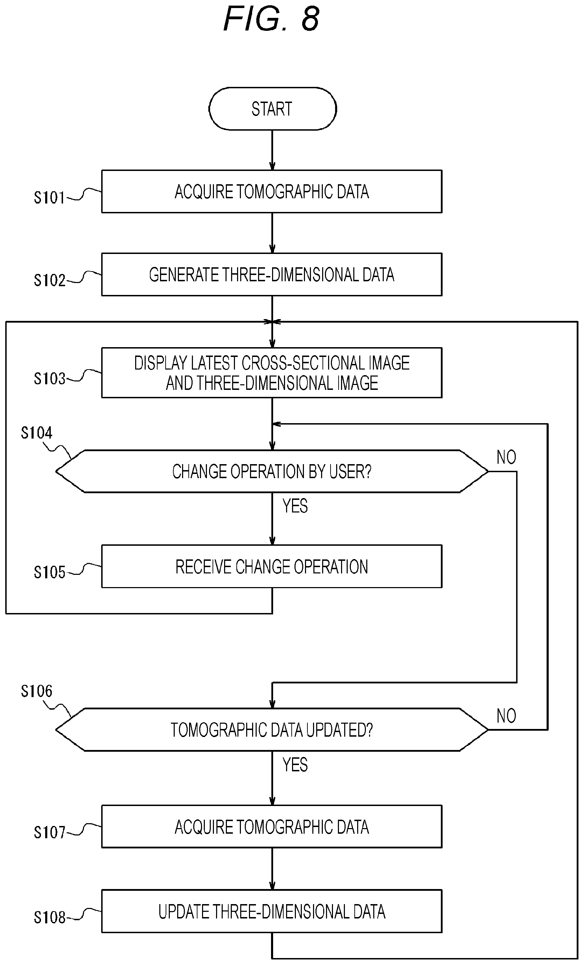

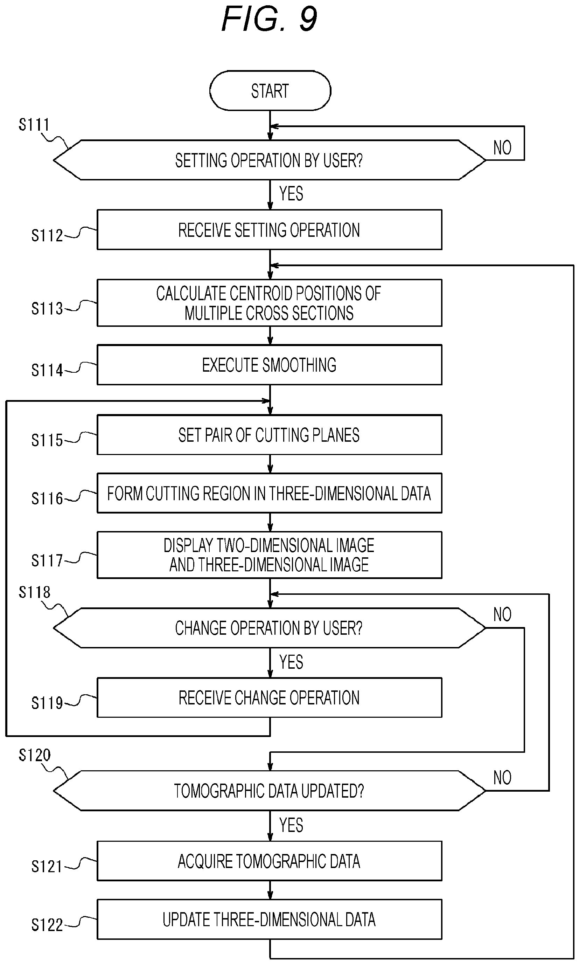

The operation of the image processing system 10 according to the present embodiment will be described with reference to Figs. 8 and 9. The operation of the image processing system 10 corresponds to an image display method according to the present embodiment.

Before a start of the procedure of Fig. 8, the probe 20 is primed by the user. Thereafter, the probe 20 is fitted into the probe connection portion 34 and the probe clamp unit 37 of the drive unit 13, and is connected and fixed to the drive unit 13. Then, the probe 20 is inserted to a target site in the biological tissue 60 such as the blood vessel or the heart.

In step S101, the scan switch included in the switch group 39 is pressed, and what is called a pullback operation is executed by pressing the pullback switch included in the switch group 39. The probe 20 transmits an ultrasound wave inside the biological tissue 60 by the ultrasound transducer 25 that moves backward in the axial direction by the pullback operation. The ultrasound transducer 25 radially transmits the ultrasound wave while moving inside the biological tissue 60. The ultrasound transducer 25 receives a reflected wave of the transmitted ultrasound wave. The probe 20 inputs a signal of the reflected wave received by the ultrasound transducer 25 to the image processing device 11. The control unit 41 of the image processing device 11 processes the input signal to sequentially generate cross-sectional images of the biological tissue 60, thereby acquiring the tomographic data 51, which includes a plurality of cross-sectional images.

Specifically, the probe 20 transmits the ultrasound wave in a plurality of directions from the rotation center to the outside by the ultrasound transducer 25 while rotating the ultrasound transducer 25 in a circumferential direction and moving it in the axial direction inside the biological tissue 60. The ultrasound transducer 25 of the probe 20 receives the reflected wave from a reflecting object present in each of the plurality of directions inside the biological tissue 60. The probe 20 transmits the signal of the received reflected wave to the image processing device 11 via the drive unit 13 and the cable 12. The communication unit 43 of the image processing device 11 receives the signal transmitted from the probe 20. The communication unit 43 executes A/D conversion for the received signal. The communication unit 43 inputs the A/D-converted signal to the control unit 41. The control unit 41 processes the input signal to calculate an intensity value distribution of the reflected wave from the reflecting object present in a transmission direction of the ultrasound wave of the ultrasound transducer 25. The control unit 41 sequentially generates two-dimensional images having a luminance value distribution corresponding to the calculated intensity value distribution as the cross-sectional images of the biological tissue 60, thereby acquiring the tomographic data 51 which is a data set of the cross-sectional images. The control unit 41 stores the acquired tomographic data 51 in the storage unit 42.

In the present embodiment, the signal of the reflected wave received by the ultrasound transducer 25 corresponds to raw data of the tomographic data 51, and the cross-sectional images generated by processing the signal of the reflected wave by the image processing device 11 correspond to processed data of the tomographic data 51.

In a modification of the present embodiment, the control unit 41 of the image processing device 11 may store the signal input from the probe 20 as it is in the storage unit 42 as the tomographic data 51. Alternatively, the control unit 41 may store data indicating the intensity value distribution of the reflected wave calculated by processing the signal input from the probe 20 in the storage unit 42 as the tomographic data 51. That is, the tomographic data 51 is not limited to the data set of the cross-sectional images of the biological tissue 60, and may be data representing a cross section of the biological tissue 60 at each moving position of the ultrasound transducer 25 in any format.

In a modification of the present embodiment, an ultrasound transducer that transmits the ultrasound wave in the plurality of directions without rotating may be used instead of the ultrasound transducer 25 that transmits the ultrasound wave in the plurality of directions while rotating in the circumferential direction.

In a modification of the present embodiment, the tomographic data 51 may be acquired using OFDI or OCT instead of being acquired using IVUS. "OFDI" is an abbreviation for optical frequency domain imaging. "OCT" is an abbreviation for optical coherence tomography. In a case where OFDI or OCT is used, as a sensor that acquires the tomographic data 51 while moving in the lumen 63 of the biological tissue 60, a sensor that acquires the tomographic data 51 by emitting light in the lumen 63 of the biological tissue 60 is used instead of the ultrasound transducer 25 that acquires the tomographic data 51 by transmitting the ultrasound wave in the lumen 63 of the biological tissue 60.

In a modification of the present embodiment, instead of the image processing device 11 generating the data set of the cross-sectional images of the biological tissue 60, another device may generate a similar data set, and the image processing device 11 may acquire the data set from the other device. That is, instead of the control unit 41 of the image processing device 11 processing the IVUS signal to generate the cross-sectional images of the biological tissue 60, another device may process the IVUS signal to generate the cross-sectional images of the biological tissue 60 and input the generated cross-sectional images to the image processing device 11.

In step S102, the control unit 41 of the image processing device 11 generates the three-dimensional data 52 of the biological tissue 60 based on the tomographic data 51 acquired in step S101. That is, the control unit 41 generates the three-dimensional data 52 based on the tomographic data 51 acquired by the sensor. Note that at this time, if already generated three-dimensional data 52 is present, it is preferable to update only data at a location corresponding to the updated tomographic data 51, instead of regenerating all pieces of the three-dimensional data 52 from the beginning. Accordingly, the data processing amount in generating the three-dimensional data 52 can be reduced, and the real-time property of the three-dimensional image 53 in the subsequent step S103 can be improved.

Specifically, the control unit 41 of the image processing device 11 generates the three-dimensional data 52 of the biological tissue 60 by stacking the cross-sectional images of the biological tissue 60 included in the tomographic data 51 stored in the storage unit 42, and converting the same into three-dimensional data. As a method of three-dimensional conversion, any method among a rendering method such as surface rendering or volume rendering, and various types of processing such as texture mapping including environment mapping, and bump mapping, which are associated with the rendering method, can be used. The control unit 41 stores the generated three-dimensional data 52 in the storage unit 42.

When the elongated medical instrument 67 such as a catheter different from the IVUS catheter or the like is inserted into the biological tissue 60, the tomographic data 51 includes data of the elongated medical instrument 67, similarly to the data of the biological tissue 60. Therefore, in step S102, the three-dimensional data 52 generated by the control unit 41 also includes the data of the elongated medical instrument 67 similarly to the data of the biological tissue 60.

The control unit 41 of the image processing device 11 classifies pixel groups of the cross-sectional images included in the tomographic data 51 acquired in step S101 into two or more classes. These two or more classes include at least a class of "tissue" to which the biological tissue 60 belongs and a class of "medical instrument" to which the elongated medical instrument 67 belongs, and may further include a class of "blood cell", a class of "indwelling object" of an indwelling stent or the like, or a class of "lesion" of lime, plaque, or the like. As a classification method, any method may be used, but in the present embodiment, a method of classifying the pixel groups of the cross-sectional images by a trained model is used. The trained model is trained by performing machine learning in advance so that the trained model can detect a region corresponding to each class from a cross-sectional image of IVUS as a sample.

In step S103, the control unit 41 of the image processing device 11 causes the display 16 to display the three-dimensional data 52 generated in step S102 as the three-dimensional image 53. At this time, the control unit 41 may set an angle for displaying the three-dimensional image 53 to any angle. The control unit 41 causes the display 16 to display the latest cross-sectional image included in the tomographic data 51 acquired in step S101 together with the three-dimensional image 53.

Specifically, the control unit 41 of the image processing device 11 generates the three-dimensional image 53 based on the three-dimensional data 52 stored in the storage unit 42. The three-dimensional image 53 includes a three-dimensional object group including a three-dimensional object representing the biological tissue 60 and a three-dimensional object representing the elongated medical instrument 67. That is, the control unit 41 generates a three-dimensional object of the biological tissue 60 from the data of the biological tissue 60 stored in the storage unit 42, and generates a three-dimensional object of the elongated medical instrument 67 from the data of the elongated medical instrument 67 stored in the storage unit 42. The control unit 41 causes the display 16 to display the latest cross-sectional image among the cross-sectional images of the biological tissue 60 included in the tomographic data 51 stored in the storage unit 42 and the generated three-dimensional image 53 via the output unit 45.

In the present embodiment, in the three-dimensional image 53, the control unit 41 of the image processing device 11 colors voxels representing the inner surface 61 of the biological tissue 60 in the first voxel group 54a corresponding to the cross section 64 indicated by the tomographic data 51 newly acquired by the sensor distinguishably from the voxel groups 55 corresponding to other cross sections of the biological tissue 60. Specifically, as illustrated in Fig. 2, the control unit 41 sets the color of the voxels representing the inner surface 61 of the biological tissue 60 in the first voxel group 54a to a color different from any color of the other voxel groups 55, thereby coloring the voxels representing the inner surface 61 of the biological tissue 60 in the first voxel group 54a distinguishably from the other voxel groups 55. For example, in the three-dimensional image 53, the control unit 41 sets the color of the voxels representing the inner surface 61 of the biological tissue 60 in the first voxel group 54a to white.

In a modification of the present embodiment, as illustrated in Fig. 6, the control unit 41 of the image processing device 11 may color all the voxels representing the biological tissue 60 in the first voxel group 54a distinguishably from the other voxel groups 55. Specifically, the control unit 41 may set the color of all the voxels representing the biological tissue 60 in the first voxel group 54a to a color different from any color of the other voxel groups 55, thereby coloring all the voxels representing the biological tissue 60 in the first voxel group 54a distinguishably from the other voxel groups 55.

In the present embodiment, the control unit 41 of the image processing device 11 combines the first graphic element 87a and the fifth graphic element 86 and causes the display 16 to display the first graphic element 87a and the fifth graphic element 86 together with the three-dimensional image 53. Specifically, as illustrated in Fig. 2, the control unit 41 displays a slider configured by combining the first graphic element 87a and the fifth graphic element 86 on the left part of the three-dimensional image 53 via the output unit 45. For example, the control unit 41 sets the color of the first graphic element 87a to white.

In the present embodiment, the control unit 41 of the image processing device 11 causes the display 16 to display the fifth graphic element 86 in a direction in which the long axis direction of the fifth graphic element 86 is parallel to the longitudinal direction of the lumen 63 in the three-dimensional image 53. Specifically, as illustrated in Fig. 2, the control unit 41 matches a movement range of the sensor indicated by the fifth graphic element 86 with a display range of the three-dimensional image 53 in the vertical direction of the screen 80, and matches the position of the sensor indicated by the first graphic element 87a with the position of the first voxel group 54a.

In step S104, if there is an operation of setting the angle for displaying the three-dimensional image 53 as a change operation by the user, processing of step S105 is executed. If there is no change operation by the user, processing of step S106 is executed.

In step S105, the control unit 41 of the image processing device 11 receives, via the input unit 44, the operation of setting the angle for displaying the three-dimensional image 53. The control unit 41 adjusts the angle for displaying the three-dimensional image 53 to the set angle. In step S103, the control unit 41 causes the display 16 to display the three-dimensional image 53 at the angle set in step S105.

Specifically, the control unit 41 of the image processing device 11 receives, via the input unit 44, an operation by the user of rotating the three-dimensional image 53 displayed on the display 16 by using the keyboard 14, the mouse 15, or the touch screen disposed integrally with the display 16. The control unit 41 interactively adjusts the angle for displaying the three-dimensional image 53 on the display 16 according to the operation by the user. Alternatively, the control unit 41 receives, via the input unit 44, an operation by the user of inputting a numerical value of the angle for displaying the three-dimensional image 53 by using the keyboard 14, the mouse 15, or the touch screen disposed integrally with the display 16. The control unit 41 adjusts the angle for displaying the three-dimensional image 53 on the display 16 in accordance with the input numerical value.

In step S106, if the tomographic data 51 is updated, processing of steps S107 and S108 is executed. If the tomographic data 51 is not updated, the presence or absence of the change operation by the user is confirmed again in step S104.

In step S107, similarly to the processing in step S101, the control unit 41 of the image processing device 11 processes the signal input from the probe 20 to newly generate cross-sectional images of the biological tissue 60, thereby acquiring the tomographic data 51 including at least one new cross-sectional image.

In step S108, the control unit 41 of the image processing device 11 updates the three-dimensional data 52 of the biological tissue 60 based on the tomographic data 51 acquired in step S107. That is, the control unit 41 updates the three-dimensional data 52 based on the tomographic data 51 acquired by the sensor. Then, in step S103, the control unit 41 causes the display 16 to display the three-dimensional data 52 updated in step S108 as the three-dimensional image 53. The control unit 41 causes the display 16 to display the latest cross-sectional image included in the tomographic data 51 acquired in step S107 together with the three-dimensional image 53. In step S108, it is preferable to update only data at a location corresponding to the updated tomographic data 51. Accordingly, the data processing amount in generating the three-dimensional data 52 can be reduced, and the real-time property of the three-dimensional image 53 can be improved in step S108.

In step Sill, if there is an operation of setting the cutting region 62 as a setting operation by the user, the processing of step S112 is executed.

In step S112, the control unit 41 of the image processing device 11 receives, via the input unit 44, the operation of setting the cutting region 62.

Specifically, the control unit 41 of the image processing device 11 receives an operation of setting the region 65 corresponding to the cutting region 62 with respect to the cross-sectional image displayed on the display 16 in step S103 via the input unit 44. In the present embodiment, the control unit 41 receives an operation of setting the two straight lines L1 and L2 extending from one point M in the cross-sectional image as the operation of setting the region 65 corresponding to the cutting region 62.

More specifically, the control unit 41 of the image processing device 11 receives, via the input unit 44, an operation by the user of designating the base angle and the opening angle on the operation panel 81 as illustrated in Fig. 2 using the keyboard 14, the mouse 15, or the touch screen disposed integrally with the display 16. That is, as the operation of setting the two straight lines L1 and L2, the control unit 41 receives an operation of designating the direction of the straight line L1 of the two straight lines L1 and L2 and the angle formed by the two straight lines L1 and L2. Here, it is assumed that the check box 85 of the operation panel 81 is in a checked state, that is, use of a centroid is selected.

In a modification of the present embodiment, the control unit 41 of the image processing device 11 may receive, via the input unit 44, an operation by the user of drawing the two straight lines L1 and L2 on a cross-sectional image displayed on the display 16 by using the keyboard 14, the mouse 15, or the touch screen disposed integrally with the display 16. That is, the control unit 41 may receive an operation of drawing the two straight lines L1 and L2 on the cross-sectional image as the operation of setting the two straight lines L1 and L2.

In step S113, the control unit 41 of the image processing device 11 calculates the centroid positions of the plurality of lateral cross sections of the lumen 63 of the biological tissue 60 by using the latest three-dimensional data 52 stored in the storage unit 42. The latest three-dimensional data 52 is the three-dimensional data 52 generated in step S102 if the processing in step S108 is not executed, and is the three-dimensional data 52 updated in step S108 if the processing in step S108 is executed. Note that at this time, if already generated three-dimensional data 52 is present, it is preferable to update only data at a location corresponding to the updated tomographic data 51, instead of regenerating all of the three-dimensional data 52 from the beginning. Accordingly, the data processing amount in generating the three-dimensional data 52 can be reduced, and the real-time property of the three-dimensional image 53 in the subsequent step S117 can be improved.

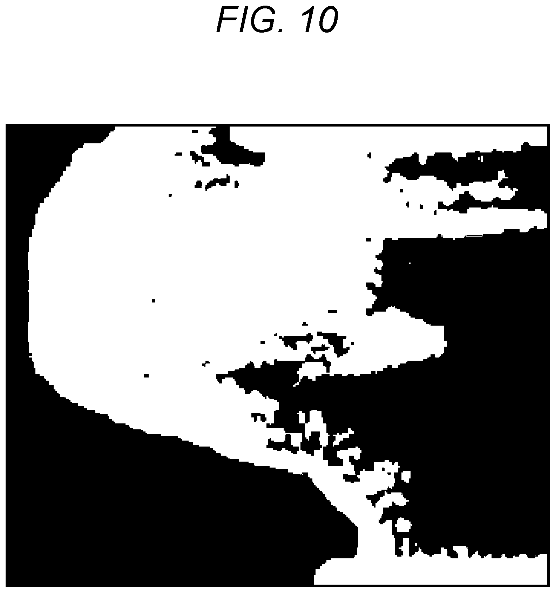

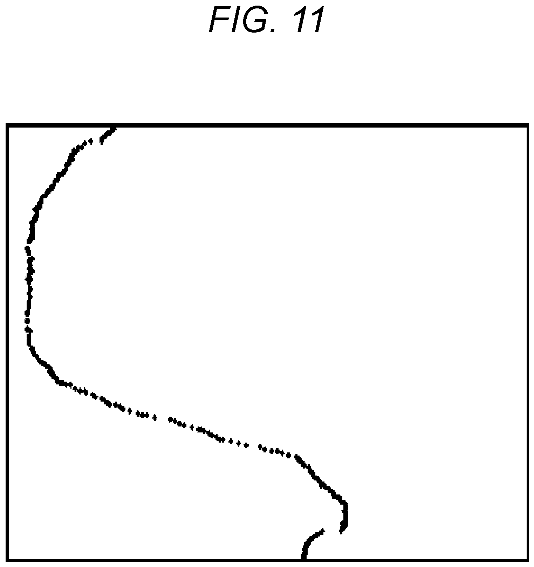

Specifically, as illustrated in Fig. 10, if the control unit 41 of the image processing device 11 generates a corresponding new cross-sectional image in step S107 for each of the plurality of cross-sectional images generated in step S101, the control unit 41 replaces each of the plurality of cross-sectional images generated in step S101 with the new cross-sectional image, and then binarizes the cross-sectional image. As illustrated in Fig. 11, the control unit 41 extracts a point cloud on the inner surface of the biological tissue 60 from the binarized cross-sectional image. For example, the control unit 41 extracts a point cloud on an inner surface of a blood vessel by extracting points corresponding to an inner surface of a main blood vessel one by one along a longitudinal direction of the cross-sectional image with an r-axis as a horizontal axis and a $\theta$-axis as a vertical axis. The control unit 41 may simply obtain the centroid of the extracted point cloud on the inner surface, but in that case, since the point cloud is not uniformly sampled over the inner surface, a centroid position shifts. Therefore, in the present embodiment, the control unit 41 calculates the convex hull of the extracted point cloud on the inner surface, and calculates a centroid position $C_n = (C_x, C_y)$ by using a formula for obtaining the centroid of a polygon as follows. However, in the following formula, it is assumed that n vertices $(x_0, y_0)$, $(x_1, y_1)$, ..., $(x_{n-1}, y_{n-1})$ are present on the convex hull counterclockwise as the point cloud on the inner surface as illustrated in Fig. 11, and $(x_n, y_n)$ is regarded as $(x_0, y_0)$.

[Math. 1]

$$C_x = \frac{1}{6A} \sum_{i=0}^{n-1} (x_i + x_{i+1})(x_i y_{i+1} - x_{i+1} y_i)$$

$$C_y = \frac{1}{6A} \sum_{i=0}^{n-1} (y_i + y_{i+1})(x_i y_{i+1} - x_{i+1} y_i)$$

$$A = \frac{1}{2} \sum_{i=0}^{n-1} (x_i y_{i+1} - x_{i+1} y_i)$$

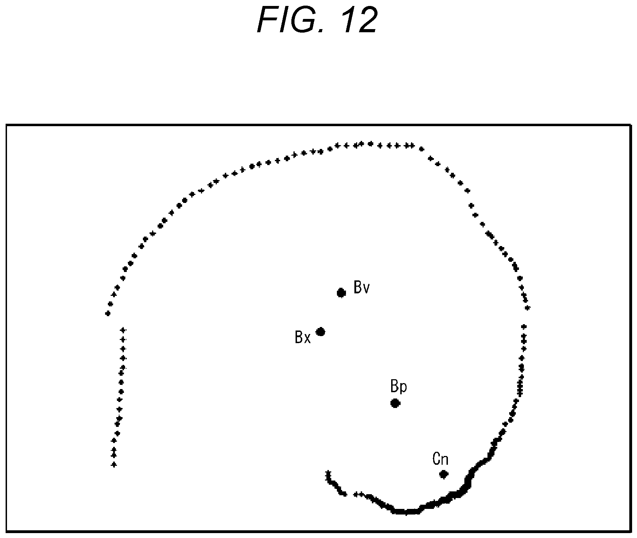

The centroid positions obtained as results are illustrated in Fig. 12. In Fig. 12, a point Cn is the center of the cross-sectional image. A point Bp is a centroid of the point cloud on the inner surface. A point Bv is a centroid of the vertices of the polygon. A point Bx is a centroid of the polygon serving as a convex hull.

As a method of calculating the centroid position of the blood vessel, a method other than the method of calculating the centroid position of the polygon serving as the convex hull may be used. For example, with respect to an original cross-sectional image that is not binarized, a method of calculating a center position of the maximum circle that falls within the main blood vessel as the centroid position may be used. Alternatively, with respect to the binarized cross-sectional image having the r-axis as the horizontal axis and the θ-axis as the vertical axis, a method of calculating an average position of pixels in a main blood vessel region as the centroid position may be used. The same method as described above may also be used when the biological tissue 60 is not a blood vessel.

In step S114, the control unit 41 of the image processing device 11 smooths calculation results of the centroid positions in step S113.

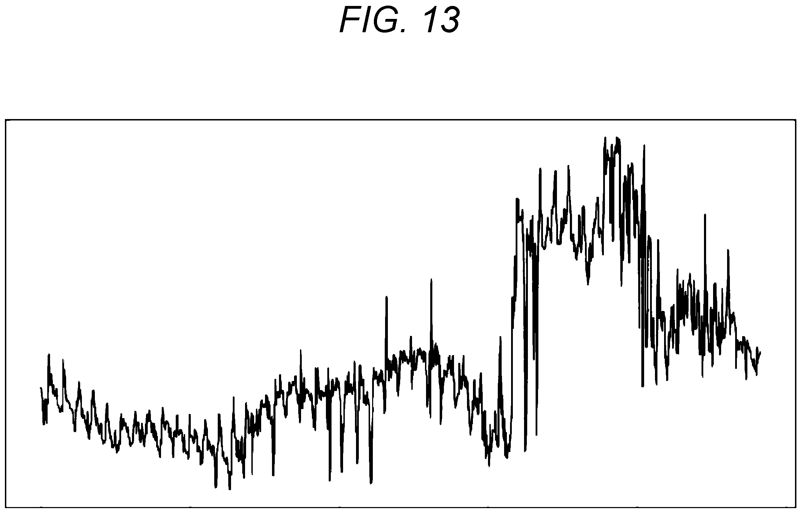

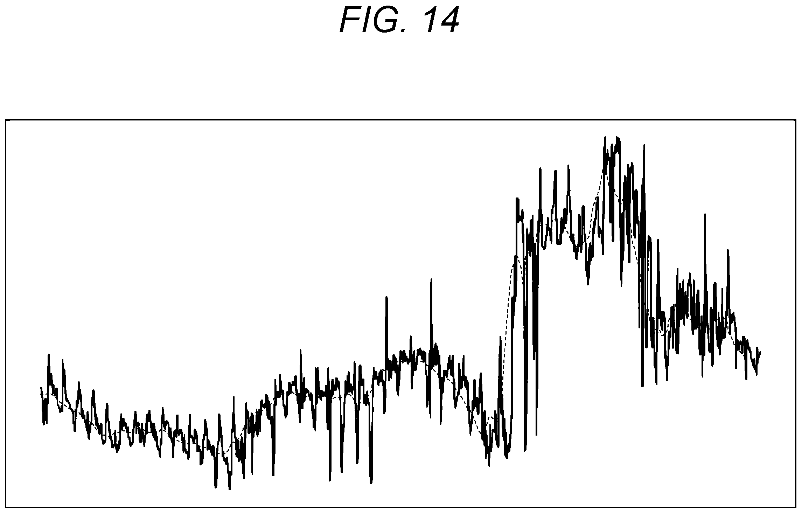

As illustrated in Fig. 13, when the calculation results of the centroid positions are viewed as a time function, it can be seen that an influence of pulsation is large. Therefore, in the present embodiment, the control unit 41 of the image processing device 11 smooths the calculation results of the centroid positions by using moving averages as indicated by a broken line in Fig. 14.

As a smoothing method, a method other than the movement average may be used. For example, exponential smoothing method, kernel method, local regression, Ramer-Douglas-Peucker algorithm, Savitzky-Golay method, smoothed spline, or SGM may be used. Alternatively, a method of executing the fast Fourier transform and then removing a highfrequency component may be used. Alternatively, Kalman filter or a low-pass filter such as Butterworth filter, Chebyshev filter, digital filter, elliptical filter, or KZ filter may be used. "SGM" is an abbreviation for stretched grid method. "KZ" is an abbreviation for Kolmogorov-Zurbenko.

Simple smoothing may cause the centroid positions to enter the tissue. In this case, the control unit 41 may divide the calculation results of the centroid positions, in the longitudinal direction of the lumen 63 of the biological tissue 60, according to positions of the plurality of lateral cross sections of the lumen 63 of the biological tissue 60, and may smooth each of the divided calculation results. That is, when a curve of the centroid positions as indicated by the broken line in Fig. 14 overlaps a tissue region, the control unit 41 may divide the curve of the centroid positions into a plurality of sections and execute individual smoothing for each section. Alternatively, the control unit 41 may adjust a degree of smoothing to be executed on the calculation results of the centroid positions according to the positions of the plurality of lateral cross sections of the lumen 63 of the biological tissue 60 in the longitudinal direction of the lumen 63 of the biological tissue 60. That is, when the curve of the centroid positions as indicated by the broken line in Fig. 14 overlaps the tissue region, the control unit 41 may decrease the degree of smoothing to be executed for a part of the sections including the overlapping points.

In step S115, as illustrated in Fig. 4, the control unit 41 of the image processing device 11 sets two planes intersecting at the single line Lb passing through the centroid positions calculated in step S113, as cutting planes P1 and P2. In the present embodiment, the control unit 41 smooths the calculation results of the centroid positions in step S114, and then sets the cutting planes P1 and P2, but the processing of step S114 may be omitted.

Specifically, the control unit 41 of the image processing device 11 sets a curve of the centroid positions obtained as a result of the smoothing in step S114 as the line Lb. The control unit 41 sets two planes intersecting at the set line Lb and including, respectively, the two straight lines L1 and L2 set in step S112 as the cutting planes P1 and P2. The control unit 41 identifies three-dimensional coordinates intersecting with the cutting planes P1 and P2 of the biological tissue 60 in the latest three-dimensional data 52 stored in the storage unit 42 as the three-dimensional coordinates of the edges of the opening exposing the lumen 63 of the biological tissue 60 in the three-dimensional image 53. The control unit 41 stores the identified three-dimensional coordinates in the storage unit 42.

In step S116, the control unit 41 of the image processing device 11 forms, in the three-dimensional data 52, a region interposed between the cutting planes P1 and P2 and exposing the lumen 63 of the biological tissue 60 in the three-dimensional image 53, as a cutting region 62.

Specifically, the control unit 41 of the image processing device 11 sets a portion identified by the three-dimensional coordinates stored in the storage unit 42 in the latest three-dimensional data 52 stored in the storage unit 42 to be hidden or transparent when the three-dimensional image 53 is displayed on the display 16. That is, the control unit 41 forms the cutting region 62 in accordance with the region 65 set in step S112.

In step S117, the control unit 41 of the image processing device 11 causes the display 16 to display the three-dimensional data 52 in which the cutting region 62 is formed in step S116 as the three-dimensional image 53. The control unit 41 causes the display 16 to display the two-dimensional image 56 representing the cross section 64 indicated by the tomographic data 51 newly acquired by the sensor and represented by the cross-sectional image displayed on the display 16 in step S103 and the region 65 corresponding to the cutting region 62 in the cross section 64 together with the three-dimensional image 53.

Specifically, the control unit 41 of the image processing device 11 generates the two-dimensional image 56 as illustrated in Fig. 2 by processing the latest cross-sectional image among the cross-sectional images of the biological tissue 60 included in the tomographic data 51 stored in the storage unit 42. The control unit 41 generates the three-dimensional image 53 as illustrated in Fig. 2 in which a portion identified by the three-dimensional coordinates stored in the storage unit 42 is hidden or transparent. The control unit 41 causes the display 16 to display the generated two-dimensional image 56 and three-dimensional image 53 via the output unit 45.

In the present embodiment, as illustrated in Fig. 2, the control unit 41 of the image processing device 11 generates, as the two-dimensional image 56, an image showing the region 65 corresponding to the cutting region 62 in a color different from that of the remaining region. For example, it is conceivable to change a white portion in a typical IVUS image to red in the region 65.

In step S118, if there is an operation of setting the cutting region 62 as a change operation by the user, the processing of step S119 is executed. If there is no change operation by the user, processing of step S120 is executed.

In step S119, the control unit 41 of the image processing device 11 receives, via the input unit 44, the operation of setting the cutting region 62, similarly to the processing in step S112. Then, the processing in and after step S115 is executed.

If the tomographic data 51 is updated in step S120, the processing in steps S121 and S122 is executed. If the tomographic data 51 is not updated, the presence or absence of the change operation by the user is confirmed again in step S118.

In step S121, similarly to the processing in step S101 or step S107, the control unit 41 of the image processing device 11 processes the signal input from the probe 20 to newly generate cross-sectional images of the biological tissue 60, thereby acquiring the tomographic data 51 including at least one new cross-sectional image.

In step S122, the control unit 41 of the image processing device 11 updates the three-dimensional data 52 of the biological tissue 60 based on the tomographic data 51 acquired in step S121. Thereafter, the processing in and after step S113 is executed. In step S122, it is preferable to update only data at a location corresponding to the updated tomographic data 51. Accordingly, the data processing amount in generating the three-dimensional data 52 can be reduced, and the real-time property of data processing in and after step S113 can be improved.

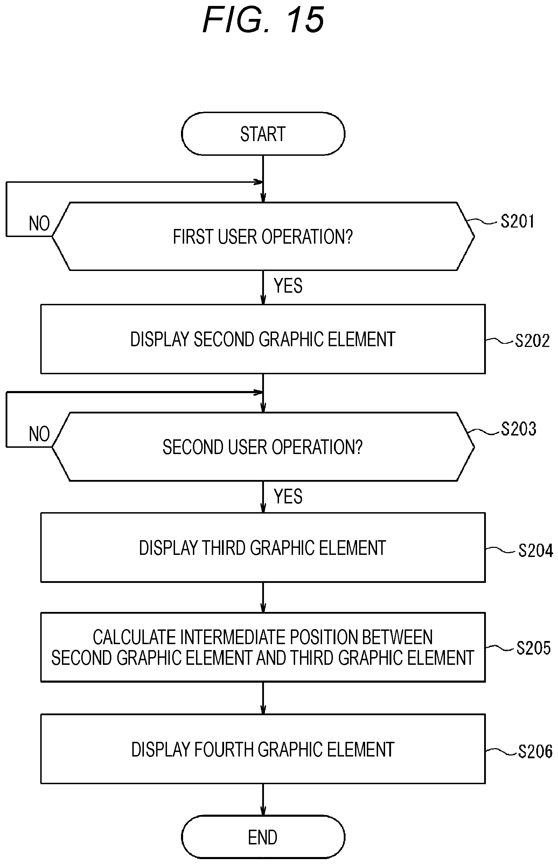

The operation of the image processing system 10 according to the present embodiment will be further described with reference to Fig. 15.

In step S201, when a marking operation that is a user operation for requesting marking of the position of the sensor is performed, the control unit 41 of the image processing device 11 receives the marking operation via the input unit 44. The marking operation is, for example, an operation of simultaneously pressing the Ctrl key and "B" on the keyboard 14, but may be an operation of clicking the first graphic element 87a with the mouse 15 while pressing the Ctrl key on the keyboard 14, or an operation of tapping the first graphic element 87a on a touch screen disposed integrally with the display 16 while pressing the Ctrl key on the keyboard 14. An erroneous operation can be prevented by including an operation of pressing one or more predetermined keys such as the Ctrl key in the marking operation.

In step S202, the control unit 41 of the image processing device 11 causes the display 16 to display the second element together with the first element, the second element being fixed at the same position as the position of the first element at the time of the marking operation. Specifically, the control unit 41 combines a graphic element group, which is an element group including the first element and the second element, and an elongated graphic element indicating a movement range of the sensor, and causes the display 16 to display the graphic element group and the elongated graphic element via the output unit 45. More specifically, the control unit 41 disposes the second graphic element 87b having a fixed position as the second element on the slider that is a combination of the first graphic element 87a having a variable position and the elongated fifth graphic element 86 as the first element. If the sensor detects the upper end of the oval fossa 66 and the marking operation is performed, the control unit 41 matches the position of the upper end of the oval fossa 66 indicated by the second graphic element 87b with the position of the second voxel group 54b.

In the present embodiment, the control unit 41 of the image processing device 11 sets the color of the second element to a color different from the color of the first element. For example, the control unit 41 sets the color of the second graphic element 87b to green. In the three-dimensional image 53, the control unit 41 also sets the color of the voxels representing the inner surface 61 of the biological tissue 60 in the second voxel group 54b to green.

In step S203, when the marking operation is performed again, the control unit 41 of the image processing device 11 receives the marking operation via the input unit 44 again.

In step S204, the control unit 41 of the image processing device 11 causes the display 16 to display the third element together with the first element and the second element, the third element being fixed at the same position as the position of the first element at the time of the marking operation performed again. Specifically, the control unit 41 adds the third element to the graphic element group displayed on the display 16 in combination with the elongated graphic element indicating the movement range of the sensor. More specifically, the control unit 41 disposes the third graphic element 87c having a fixed position as the third element on the slider that is a combination of the first graphic element 87a having a variable position and the elongated fifth graphic element 86 as the first element. If the sensor detects the lower end of the oval fossa 66 and the marking operation is performed again, the control unit 41 matches the position of the lower end of the oval fossa 66 indicated by the third graphic element 87c with the position of the third voxel group 54c.

In the present embodiment, the control unit 41 of the image processing device 11 sets the color of the third element to a color different from the color of the second element. For example, the control unit 41 sets the color of the third graphic element 87c to red. In the three-dimensional image 53, the control unit 41 also sets the color of the voxels representing the inner surface 61 of the biological tissue 60 in the third voxel group 54c to red.

In step S205, the control unit 41 of the image processing device 11 calculates the intermediate position between the second element and the third element. Specifically, the control unit 41 calculates the intermediate position between the second graphic element 87b and the third graphic element 87c.

In step S206, the control unit 41 of the image processing device 11 causes the display 16 to display the fourth element together with the first element, the second element, and the third element, the fourth element being fixed at the position calculated in step S205. Specifically, the control unit 41 adds the fourth element to the graphic element group displayed on the display 16 in combination with the elongated graphic element indicating the movement range of the sensor. More specifically, the control unit 41 disposes the fourth graphic element 87d having a fixed position as the fourth element on the slider that is a combination of the first graphic element 87a having a variable position and the elongated fifth graphic element 86 as the first element. If the sensor detects the upper end of the oval fossa 66 and the first marking operation is performed, and the sensor detects the lower end of the oval fossa 66 and the second marking operation is performed, the control unit 41 matches the intermediate position of the upper end and the lower end of the oval fossa 66 indicated by the fourth graphic element 87d with the position of the fourth voxel group 54d.

In the present embodiment, the control unit 41 of the image processing device 11 sets the color of the fourth element to a color different from the colors of the second element and the third element. For example, the control unit 41 sets the color of the fourth graphic element 87d to yellow. In the three-dimensional image 53, the control unit 41 also sets the color of the voxels representing the inner surface 61 of the biological tissue 60 in the fourth voxel group 54d to yellow.

In step S205, any position other than the intermediate position between the second element and the third element may be calculated as long as the position is between the second element and the third element. For example, a position separated from the second graphic element 87b by a predetermined distance or a position separated from the third graphic element 87c by a predetermined distance may be calculated between the second graphic element 87b and the third graphic element 87c.

The three graphic elements additionally displayed in steps S201 to S206 may be erased by performing an erasing operation which is an operation different from the marking operation. The erasing operation is, for example, an operation of simultaneously pressing the Ctrl key and "D" on the keyboard 14, but may be an operation of clicking each graphic element with the mouse 15 while pressing the Ctrl key on the keyboard 14, or an operation of tapping each graphic element on a touch screen disposed integrally with the display 16 while pressing the Ctrl key on the keyboard 14. When the Ctrl key and "D" are pressed simultaneously, the three graphic elements may be erased at a time.

According to the present embodiment, the procedure can be performed in the following five steps.

1. The pullback unit is operated to search for the oval fossa 66.
2. The pullback unit is operated to mark the upper end and the lower end of the oval fossa 66. A more accurate position can be identified by determining the upper and lower ends with reference to the oval fossa 66 in the three-dimensional image 53.
3. The pullback unit is moved according to the information of the upper end and the lower end to a certain position therebetween.
4. The septal puncture needle is operated to bring the distal end into contact with the plane where the current ultrasound element is located. At that time, an X-ray device or ultrasonic two-dimensional information may be used in combination.
5. Whether or not the distal end is in contact with the wall surface on the target plane correctly is confirmed by confirming the three-dimensional model of the elongated medical instrument 67 included in the three-dimensional image 53, and puncture is performed. Before this step, a target plane may be defined in advance, and marking may be performed.

In the present embodiment, the marking operation is received up to twice, but in a modification of the present embodiment, the marking operation may be received only once. That is, when the second element is displayed on the display 16, the marking operation may not be accepted. Alternatively, the marking operation may be received three or more times. That is, even when the second element and the third element are being displayed on the display 16, the third and subsequent marking operations may be received, and an additional element fixed at the same position as the position of the first element at the time of each marking operation may be displayed on the display 16.

In a modification of the present embodiment, the processing of steps S205 and S206 may be omitted. That is, the fourth element may not be displayed.

In a modification of the present embodiment, the control unit 41 of the image processing device 11 may calculate the distance from a certain reference position to the position of the upper end of the oval fossa 66. The calculated distance may be displayed in the screen 80, for example, in the vicinity of the second graphic element 87b. The control unit 41 may further calculate the distance from the reference position to the position of the lower end of the oval fossa 66. The calculated distance may be displayed in the screen 80, for example, in the vicinity of the third graphic element 87c. The control unit 41 may calculate the distance from the reference position to the position calculated in step S205. The calculated distance may be displayed in the screen 80, for example, in the vicinity of the fourth graphic element 87d.

In a modification of the present embodiment, the control unit 41 of the image processing device 11 may set a color of a region between a cross section corresponding to the position of the second element and a cross section corresponding to the position of the third element to a color different from a color of an adjacent region in a three-dimensional image 53. That is, in the three-dimensional image 53, the control unit 41 may set a color of the voxel groups existing between the second voxel group 54b and the third voxel group 54c to a color different from the default color. Note that the voxel groups to be set to a different color may be only a voxel group representing the inner surface 61 or a voxel group adjacent to the voxel group representing the inner surface 61 and representing the lumen 63 existing between the second voxel group 54b and the third voxel group 54c.

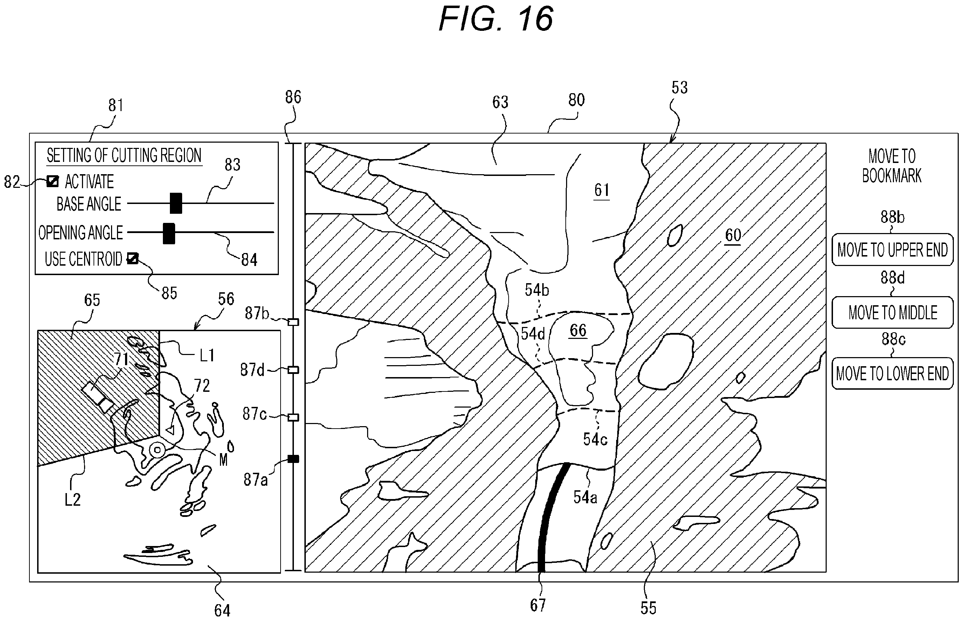

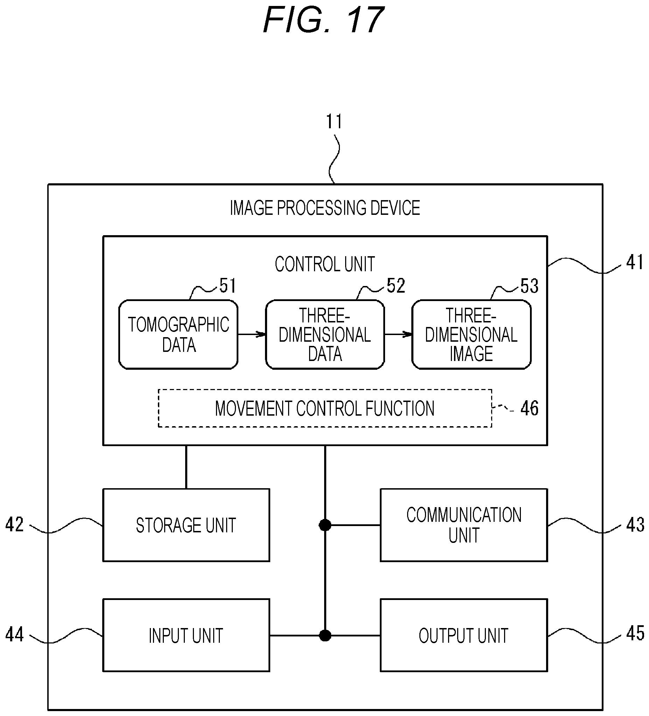

In a modification of the present embodiment, when the user clicks a button or presses a shortcut key, the pullback unit may automatically move to each bookmark position. In the example of Fig. 16, movement buttons 88b, 88c, and 88d not illustrated in the example of Fig. 2 are added to the screen 80. The movement button 88b is a button for requesting movement of the sensor to a position corresponding to the position of the second graphic element 87b, that is, the upper end of the oval fossa 66. The movement button 88c is a button for requesting movement of the sensor to a position corresponding to the position of the third graphic element 87c, that is, the lower end of the oval fossa 66. The movement button 88d is a button for requesting movement of the sensor to a position corresponding to the position of the fourth graphic element 87d, that is, an intermediate position between the upper end and the lower end of the oval fossa 66. In this modification, as illustrated in Fig. 17, the control unit 41 of the image processing device 11 includes a movement control function 46 that receives an operation of requesting movement of the sensor via the input unit 44 and moves the sensor. The movement control function 46 is a function of controlling the movement of the pullback unit via the communication unit 43 of the image processing device 11. The control unit 41 moves the sensor to a position according to a request from the user by moving the pullback unit using the movement control function 46. For example, when the user clicks the movement button 88b, the control unit 41 moves the sensor to the upper end of the oval fossa 66. That is, the control unit 41 moves the sensor to a position corresponding to a position of the second element upon receiving an operation of requesting movement of the sensor to a position corresponding to the position of the second element. Alternatively, when the user clicks the movement button 88c, the control unit 41 moves the sensor to the lower end of the oval fossa 66. That is, the control unit 41 moves the sensor to a position corresponding to the position of the third element upon receiving an operation of requesting movement of the sensor to a position corresponding to the position of the third element. Alternatively, when the user clicks the movement button 88d, the control unit 41 moves the sensor to the intermediate position between the upper end and the lower end of the oval fossa 66. That is, the control unit 41 moves the sensor to a position corresponding to the position of the fourth element upon receiving an operation of requesting movement of the sensor to the position corresponding to the position of the fourth element.

The present disclosure is not limited to the above-described embodiment. For example, two or more blocks illustrated in the block diagram may be integrated, or one block may be divided. Instead of executing two or more steps described in the flowchart in time series according to the description, the steps may be executed in parallel or in a different order according to the processing capability of the device that executes each step or as necessary. In addition, modifications can be made without departing from the gist of the present disclosure.

Hereinafter, a second embodiment as another embodiment of the present disclosure will be described with reference to the drawings.

In the drawings, the same or corresponding portions are denoted by the same reference numerals. In the description of the present embodiment, description of the same or corresponding parts will be omitted or simplified as appropriate.

An outline of the present embodiment will be described with reference to Figs. 1, 4, 5, and 18 to 20.

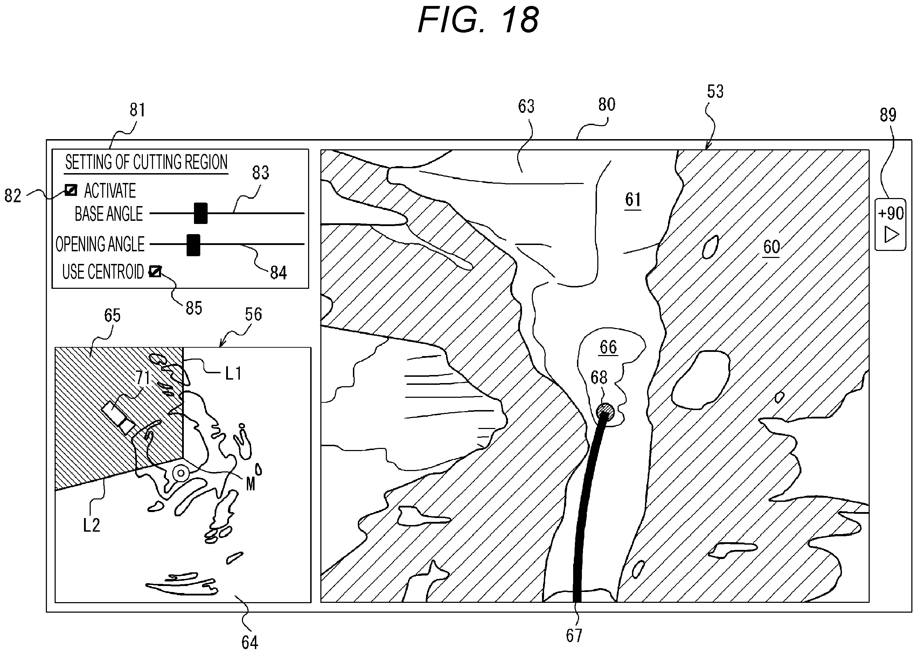

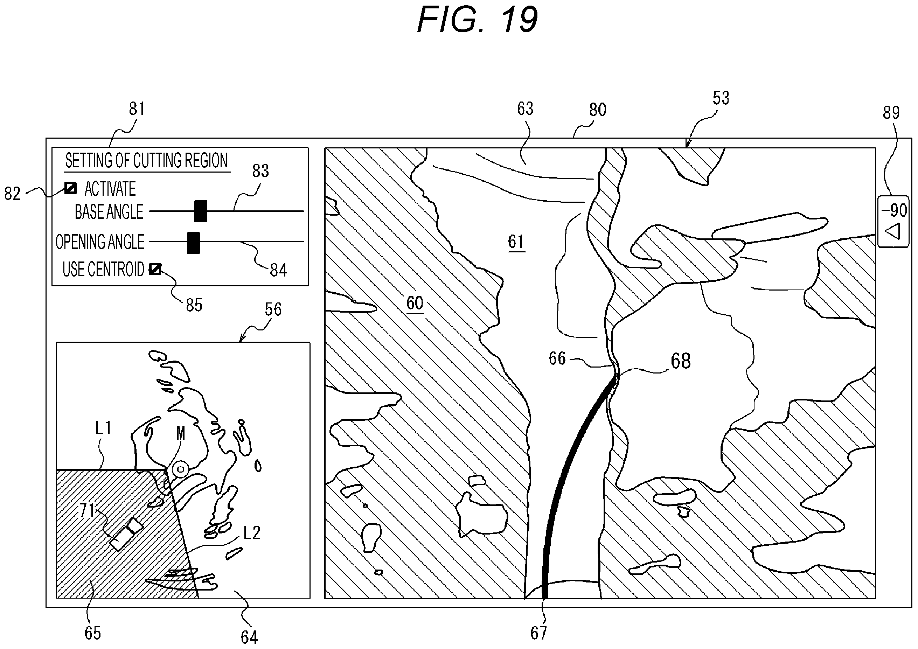

The image processing device 11 according to the present embodiment is a computer that causes the display 16 to display the three-dimensional data 52 representing the biological tissue 60 as the three-dimensional image 53. As illustrated in Fig. 4, the image processing device 11 forms, in the three-dimensional data 52, a cutting region 62 exposing the lumen 63 of the biological tissue 60 on the three-dimensional image 53. The image processing device 11 adjusts the viewpoint for displaying the three-dimensional image 53 on the display 16 according to the position of the cutting region 62. The viewpoint is a position of a virtual camera 71 disposed in a three-dimensional space. The image processing device 11 changes, upon receiving the user operation of requesting rotation of the viewpoint, the position of the cutting region 62 from the first position at the time of the user operation to the second position after rotation around the rotation axis passing through the reference point located in the lumen 63 on the reference plane and extending in the direction perpendicular to the reference plane, the reference plane extending in the horizontal direction in the three-dimensional image 53 and including the viewpoint. The horizontal direction is an XY direction illustrated in Fig. 4. The direction perpendicular to the reference plane is the Z direction illustrated in Fig. 4. The reference point may be any point located in the lumen 63 on the reference plane, such as the center point of the IVUS catheter, but in the present embodiment, it is the centroid of the lumen 63 on the reference plane. In the example of Fig. 4, when the cross section C1 of the biological tissue 60 overlaps the reference plane, the corresponding centroid B1 becomes the reference point. The same applies to the cross sections C2, C3, and C4 of the biological tissue 60 and the corresponding centroids B2, B3, and B4. As illustrated in Figs. 18 and 19, the image processing device 11 rotates the viewpoint around the rotation axis according to the second position.

According to the present embodiment, usefulness for confirming a position in the biological tissue 60 is improved. For example, when a procedure such as ablation using IVUS is performed, a doctor performing the procedure while operating the catheter or a user such as a clinical engineer operating the IVUS system while viewing the display 16 can rotate the viewpoint around the rotation axis by performing a specific user operation. For example, when the septal paracentesis is performed, a transition from a state in which the oval fossa 66 can be viewed from the front in the three-dimensional image 53 as illustrated in Fig. 18 to a state in which the oval fossa 66 can be viewed from the side in the three-dimensional image 53 as illustrated in Fig. 19 can be instantaneously performed by one user operation. Therefore, sufficient usefulness for confirming the puncture position is obtained.

As illustrated in Figs. 18 and 19, the image processing device 11 causes the display 16 to display the two-dimensional image 56 representing a cross section 64 of the biological tissue 60 and a region 65 corresponding to the cutting region 62 in the cross section 64 together with the three-dimensional image 53. In the two-dimensional image 56, the position of the camera 71 with respect to the cross section 64 is displayed.

According to the present embodiment, how a part of the structure of the biological tissue 60 is cut can be indicated. Therefore, the user can grasp from the two-dimensional image 56 what type of structure the portion of the biological tissue 60 that is not cut or displayed in the three-dimensional image 53 is. For example, in a case where the user is an operator, an operation on the inside of the biological tissue 60 can be easily performed.

The image processing device 11 causes the display 16 to display a three-dimensional object representing an elongated medical instrument 67 inserted into the lumen 63 so as to be included in the three-dimensional image 53. In the present embodiment, the elongated medical instrument 67 is a catheter in which a puncture needle is attached to the distal end, but may be another type of medical instrument such as a guide wire as long as it is elongated.

The biological tissue 60 includes, for example, an organ such as a blood vessel or a heart. The biological tissue 60 is not limited to only an anatomically single organ or a part thereof, but also includes a tissue including a lumen across a plurality of organs. An example of such a tissue is, specifically, a part of a vascular tissue extending from an upper part of an inferior vena cava to a lower part of a superior vena cava through a right atrium.

In Figs. 18 and 19, the operation panel 81, the two-dimensional image 56, the three-dimensional image 53, and a button 89 are displayed on the screen 80.

The operation panel 81 is a GUI component for setting the cutting region 62. "GUI" is an abbreviation for graphical user interface. The operation panel 81 includes a check box 82 for selecting whether to activate settings of the cutting region 62, a slider 83 for setting a base angle, a slider 84 for setting an opening angle, and a check box 85 for selecting whether to use a centroid.

The base angle is a rotation angle of one straight line L1 of two straight lines L1 and L2 extending from one point M in the cross-sectional image representing the cross section 64 of the biological tissue 60. Therefore, setting the base angle corresponds to setting the direction of the straight line L1. The opening angle is an angle between the two straight lines L1 and L2. Therefore, setting the opening angle corresponds to setting the angle formed by the two straight lines L1 and L2. The point M is the centroid of the cross section 64. In a case where non-use of a centroid is selected, the point M may be set to a point other than the centroid on the cross section 64.

The two-dimensional image 56 is an image obtained by processing a cross-sectional image. In the two-dimensional image 56, the color of the region 65 corresponding to the cutting region 62 is changed in order to clearly indicate which portion of the cross section 64 is cut.

In the present embodiment, the viewpoint for displaying the three-dimensional image 53 on the screen 80 is adjusted according to the position of the cutting region 62.

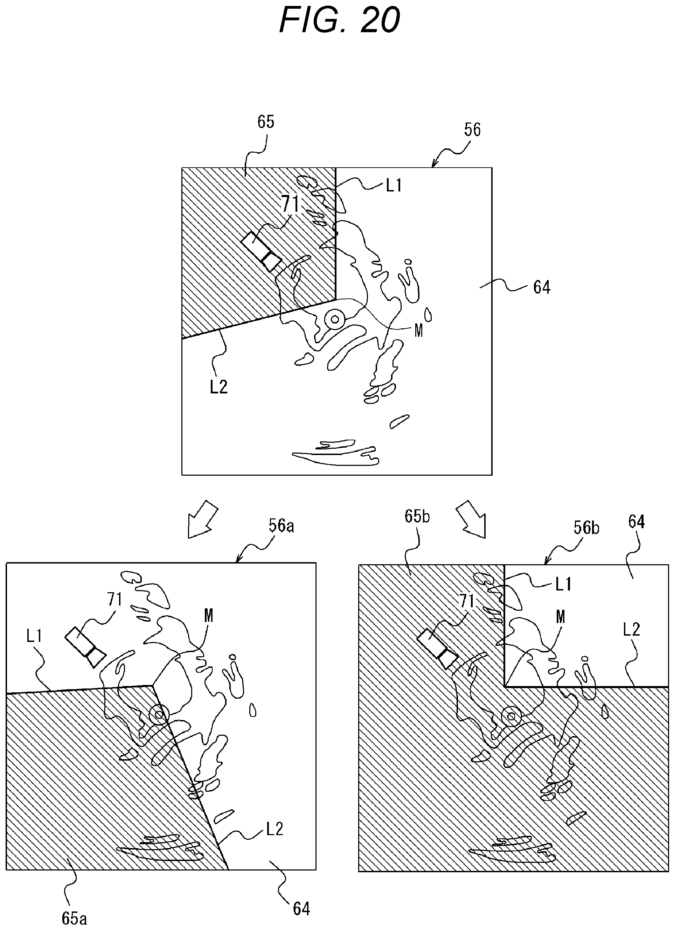

In the present embodiment, the cutting region 62 can be determined using the two-dimensional image 56. Specifically, as illustrated in Fig. 20, the position or size of the cutting region 62 can be set by adjusting the base angle or the opening angle to set the position or size of the region 65 obtained by being divided by the two straight lines L1 and L2 in the two-dimensional image 56. For example, in a case where the base angle is changed such that the straight line L1 rotates counterclockwise by about 90 degrees, a region 65a moved according to the change of the base angle is obtained in a two-dimensional image 56a. Then, the position of the cutting region 62 is adjusted according to the position of the region 65a. Alternatively, in a case where the opening angle is changed such that the angle between the two straight lines L1 and L2 is increased, a region 65b enlarged according to the change in the opening angle is obtained in a two-dimensional image 56b. Then, the size of the cutting region 62 is adjusted according to the size of the region 65b. Both the position and the size of the cutting region 62 can be set by adjusting both the base angle and the opening angle to set both the position and the size of the region 65 in the two-dimensional image 56. The position of the camera 71 may be appropriately adjusted according to the position or size of the cutting region 62.

In the present embodiment, an image corresponding to the current position of the sensor, that is, the latest image is always displayed as the two-dimensional image 56.

In a modification of the present embodiment, the base angle may be set by dragging the straight line L1 or by inputting a numerical value instead of being set by operating the slider 83. Similarly, the opening angle may be set by dragging the straight line L2 or by inputting a numerical value instead of being set by operating the slider 84.

In the three-dimensional image 53, the cutting region 62 determined using the two-dimensional image 56 is hidden or transparent.

The button 89 is a graphic element pressed to request rotation of the viewpoint. The button 89 may be displayed at any position, but are displayed on the right part of the three-dimensional image 53 in the present embodiment. As illustrated in Figs. 18 and 19, the rotation angle and the rotation direction of the viewpoint are notated on the button 89. In the present embodiment, as illustrated in Fig. 18, when the button 89 is pressed with the notation of "+90" and a triangle pointing to the right, the viewpoint is rotated by 90 degrees in the circumferential direction. Then, the three-dimensional image 53 is switched to an image as illustrated in Fig. 19. The notation on the button 89 also changes. As illustrated in Fig. 19, when the button 89 is pressed with the notation of "-90" and a triangle pointing to the left, the viewpoint is reversely rotated by 90 degrees in the circumferential direction. Then, the three-dimensional image 53 is switched to an image as illustrated in Fig. 18. The notation on the button 89 also changes. The circumferential direction is a direction of rotation around the IVUS catheter axis in the present embodiment, but may be a direction of rotation around the centroid of each cross section of the biological tissue 60.

For example, as illustrated in Fig. 18, it is possible to clearly grasp the positional relation between the oval fossa 66 and the elongated medical instrument 67 from a viewpoint in which the oval fossa 66 can be confirmed from the front. When the viewpoint is rotated by 90 degrees, as illustrated in Fig. 19, the oval fossa 66 can be confirmed from the side, and the right atrial structure, the left atrial structure, and the position where the elongated medical instrument 67 is in contact can be captured. According to the present embodiment, the three-dimensional object can be instantaneously rotated any number of times by pressing the button 89. Therefore, it is possible to easily confirm both the positional relation between the oval fossa 66 and the elongated medical instrument 67 and the right atrial structure, the left atrial structure, and the position where the elongated medical instrument 67 is in contact.

The rotation angle of the viewpoint is not limited to 90 degrees, and may be any predetermined angle. Alternatively, the rotation angle of the viewpoint may be variable. That is, the rotation angle of the viewpoint may be arbitrarily adjusted by the user.

In the present embodiment, when the button 89 is pressed, the viewpoint is instantaneously switched, but an animation in which the three-dimensional object rotates may be displayed.

In a modification of the present embodiment, the button 89 may be divided into a first button for rotating the viewpoint clockwise by 90 degrees about the IVUS catheter axis and a second button displayed simultaneously with the first button and for rotating the viewpoint counterclockwise by 90 degrees about the IVUS catheter axis.

In a modification of the present embodiment, instead of the operation of pressing the button 89, the user operation of requesting rotation of the viewpoint may be an operation of pressing one or more predetermined keys such as simultaneously pressing the Ctrl key and "R".

In Fig. 4, an X direction and a Y direction orthogonal to the X direction correspond to the lateral directions of the lumen 63 of the biological tissue 60. A Z direction orthogonal to the X direction and the Y direction corresponds to the longitudinal direction of the lumen 63 of the biological tissue 60.

In the example of Fig. 4, it is assumed that the check box 85 of the operation panel 81 is in a checked state, that is, use of a centroid is selected. The image processing device 11 calculates the positions of centroids B1, B2, B3, and B4 of respective cross sections C1, C2, C3, and C4 of the biological tissue 60 using the three-dimensional data 52. The image processing device 11 sets two planes intersecting at a single line Lb passing through the positions of the centroids B1, B2, B3, and B4 and including, respectively, the two straight lines L1 and L2 as cutting planes P1 and P2. For example, assuming that the point M illustrated in Figs. 18 and 19 is the point B3, the straight line L1 is an intersection line between the cross section C3 and the cutting plane P1, and the straight line L2 is an intersection line between the cross section C3 and the cutting plane P2. The image processing device 11 forms, in the three-dimensional data 52, a region interposed between the cutting planes P1 and P2 and exposing the lumen 63 of the biological tissue 60 in the three-dimensional image 53, as the cutting region 62.

In the case of a three-dimensional model of the biological tissue 60 bent as illustrated in Fig. 4, when the three-dimensional model is cut along a single plane to display the lumen 63, there is a case in which an inside of the biological tissue 60 cannot be correctly displayed. In the present embodiment, as illustrated in Fig. 4, by continuously capturing centroids of cross sections of the biological tissue 60, the three-dimensional model can be cut such that the inside of the biological tissue 60 can be reliably displayed.

In Fig. 4, for convenience, the four cross sections C1, C2, C3, and C4 are illustrated as a plurality of lateral cross sections of the lumen 63 of the biological tissue 60, but the number of cross sections serving as calculation targets of the centroid positions is not limited to four, and is preferably the same number as that of cross-sectional images acquired by IVUS.

In another example different from Fig. 4, it is assumed that the check box 85 of the operation panel 81 is not in a checked state, that is, non-use of a centroid is selected. In such an example, the image processing device 11 sets two planes intersecting at any one line passing through the point M, such as a straight line extending in the Z direction through the point M, and including, respectively, the two straight lines L1 and L2, as the cutting planes P1 and P2.

A configuration of an image processing system 10 according to the present embodiment will be described with reference to Fig. 1.

The image processing system 10 includes the image processing device 11, a cable 12, a drive unit 13, a keyboard 14, a mouse 15, and the display 16.

The image processing device 11 is a dedicated computer specialized for image diagnosis in the present embodiment, but may be a general-purpose computer such as a PC. "PC" is an abbreviation for personal computer.

The cable 12 is used to connect the image processing device 11 and the drive unit 13.

The drive unit 13 is a device that is used by being connected to a probe 20 illustrated in Fig. 7 and drives the probe 20. The drive unit 13 is also referred to as an MDU. "MDU" is an abbreviation for motor drive unit. The probe 20 is applied to IVUS. The probe 20 is also referred to as an IVUS catheter or an image diagnostic catheter.

The keyboard 14, the mouse 15, and the display 16 are connected to the image processing device 11 via a certain cable or wirelessly. The display 16 is, for example, an LCD, an organic EL display, or an HMD. "LCD" is an abbreviation for liquid crystal display. "EL" is an abbreviation for electro luminescence. "HMD" is an abbreviation for head-mounted display.

The image processing system 10 optionally further includes a connection terminal 17 and a cart unit 18.

The connection terminal 17 is used to connect the image processing device 11 and an external device. The connection terminal 17 is, for example, a USB terminal. "USB" is an abbreviation for universal serial bus. The external device is, for example, a recording medium such as a magnetic disc drive, a magneto-optical disc drive, or an optical disc drive.

The cart unit 18 is a cart equipped with casters for movement. The image processing device 11, the cable 12, and the drive unit 13 are disposed on a cart body of the cart unit 18. The keyboard 14, the mouse 15, and the display 16 are disposed on an uppermost table of the cart unit 18.

Configurations of the probe 20 and the drive unit 13 according to the present embodiment will be described with reference to Fig. 7.

The probe 20 includes a drive shaft 21, a hub 22, a sheath 23, an outer tube 24, an ultrasound transducer 25, and a relay connector 26.

The drive shaft 21 passes through the sheath 23 to be inserted into a lumen of a living body and the outer tube 24 connected to a proximal end of the sheath 23, and extends to an inside of the hub 22 disposed at a proximal end of the probe 20. The drive shaft 21 is provided with the ultrasound transducer 25, which transmits and receives signals, at a distal end of the drive shaft 21, and is rotatably disposed in the sheath 23 and the outer tube 24. The relay connector 26 connects the sheath 23 and the outer tube 24.

The hub 22, the drive shaft 21, and the ultrasound transducer 25 are connected to each other to integrally move forward and backward in an axial direction. Therefore, for example, when the hub 22 is pressed toward the distal side, the drive shaft 21 and the ultrasound transducer 25 move inside the sheath 23 toward the distal side. For example, when the hub 22 is pulled toward the proximal side, the drive shaft 21 and the ultrasound transducer 25 move inside the sheath 23 toward the proximal side as indicated by an arrow.

The drive unit 13 includes a scanner unit 31, a slide unit 32, and a bottom cover 33.

The scanner unit 31 is also referred to as a pullback unit. The scanner unit 31 is connected to the image processing device 11 via the cable 12. The scanner unit 31 includes a probe connection portion 34 connected to the probe 20, and a scanner motor 35 which is a drive source for rotating the drive shaft 21.

The probe connection portion 34 is freely detachably connected to the probe 20 through an insertion port 36 of the hub 22 disposed at the proximal end of the probe 20. Inside the hub 22, the proximal end of the drive shaft 21 is rotatably supported, and the rotational force of the scanner motor 35 is transmitted to the drive shaft 21. A signal is transmitted and received between the drive shaft 21 and the image processing device 11 via the cable 12. In the image processing device 11, generation of a tomographic image of a body lumen and image processing are executed based on the signal transmitted from the drive shaft 21.

The slide unit 32 is mounted with the scanner unit 31 that moves forward and backward, and is mechanically and electrically connected to the scanner unit 31. The slide unit 32 includes a probe clamp unit 37, a slide motor 38, and a switch group 39.

The probe clamp unit 37 is disposed coaxially with the probe connection portion 34 and distal of the probe connection portion 34, and supports the probe 20 to be connected to the probe connection portion 34.

The slide motor 38 is a drive source that generates a driving force in the axial direction. The scanner unit 31 moves forward and backward when driven by the slide motor 38, and the drive shaft 21 moves forward and backward in the axial direction accordingly. The slide motor 38 is, for example, a servo motor.

The switch group 39 includes, for example, a forward switch and a pullback switch that are pressed when the scanner unit 31 is to be moved forward or backward, and a scan switch that is pressed when image drawing is to be started or ended. Various switches are included in the switch group 39 as necessary without being limited to the example here.

When the forward switch is pressed, the slide motor 38 rotates forward, and the scanner unit 31 moves forward. Meanwhile, when the pullback switch is pressed, the slide motor 38 rotates backward, and the scanner unit 31 moves backward.

When the scan switch is pressed, the image drawing is started, the scanner motor 35 is driven, and the slide motor 38 is driven to move the scanner unit 31 backward. The user such as the operator connects the probe 20 to the scanner unit 31 in advance, such that the drive shaft 21 rotates and moves toward the proximal side in the axial direction upon the start of the image drawing. When the scan switch is pressed again, the scanner motor 35 and the slide motor 38 are stopped, and the image drawing is ended.

The bottom cover 33 covers the bottom and the entire circumference of a side surface on a bottom side of the slide unit 32, and can be attached to and removed from the bottom of the slide unit 32.

A configuration of the image processing device 11 will be described with reference to Fig. 5.

The image processing device 11 includes a control unit 41, a storage unit 42, a communication unit 43, an input unit 44, and an output unit 45.

The control unit 41 includes at least one processor, at least one programmable circuit, at least one dedicated circuit, or any combination thereof. The processor is a general-purpose processor such as a CPU or GPU, or a dedicated processor specialized for specific processing. "CPU" is an abbreviation for central processing unit. "GPU" is an abbreviation for graphics processing unit. The programmable circuit is, for example, an FPGA. "FPGA" is an abbreviation for field-programmable gate array. The dedicated circuit is, for example, an ASIC. "ASIC" is an abbreviation for application specific integrated circuit. The control unit 41 executes processing related to an operation of the image processing device 11 while controlling each unit of the image processing system 10 including the image processing device 11.

The storage unit 42 includes at least one semiconductor memory, at least one magnetic memory, at least one optical memory, or any combination thereof. The semiconductor memory is, for example, a RAM or a ROM. "RAM" is an abbreviation for random access memory. "ROM" is an abbreviation for read only memory. The RAM is, for example, an SRAM or a DRAM. "SRAM" is an abbreviation for static random access memory. "DRAM" is an abbreviation for dynamic random access memory. The ROM is, for example, an EEPROM. "EEPROM" is an abbreviation for electrically erasable programmable read only memory. The storage unit 42 functions as, for example, a main storage device, an auxiliary storage device, or a cache memory. The storage unit 42 stores data used for the operation of the image processing device 11, such as the tomographic data 51, and data obtained by the operation of the image processing device 11, such as the three-dimensional data 52 and the three-dimensional image 53.

The communication unit 43 includes at least one communication interface. The communication interface is, for example, a wired LAN interface, a wireless LAN interface, or an image diagnostic interface for receiving IVUS signals and executing A/D conversion for the IVUS signals. "LAN" is an abbreviation for local area network. "A/D" is an abbreviation for analog to digital. The communication unit 43 receives data used for the operation of the image processing device 11 and transmits data obtained by the operation of the image processing device 11. In the present embodiment, the drive unit 13 is connected to the image diagnostic interface included in the communication unit 43.

The input unit 44 includes at least one input interface. The input interface is, for example, a USB interface, an HDMI (registered trademark) interface, or an interface compatible with a short-range wireless communication standard such as Bluetooth (registered trademark). "HDMI (registered trademark)" is an abbreviation for high-definition multimedia interface. The input unit 44 receives an operation by the user such as an operation of inputting data used for the operation of the image processing device 11. In the present embodiment, the keyboard 14 and the mouse 15 are connected to the USB interface or the interface compatible with short-range wireless communication included in the input unit 44. When a touch screen is disposed integrally with the display 16, the display 16 may be connected to the USB interface or the HDMI (registered trademark) interface included in the input unit 44.

The output unit 45 includes at least one output interface. The output interface is, for example, a USB interface, an HDMI (registered trademark) interface, or an interface compatible with a short-range wireless communication standard such as Bluetooth (registered trademark). The output unit 45 outputs data obtained by the operation of the image processing device 11. In the present embodiment, the display 16 is connected to the USB interface or the HDMI (registered trademark) interface included in the output unit 45.

A function of the image processing device 11 is implemented by executing an image processing program according to the present embodiment by the processor corresponding to the control unit 41. That is, the function of the image processing device 11 is implemented by software. The image processing program causes a computer to function as the image processing device 11 by causing the computer to execute the operation of the image processing device 11. That is, the computer functions as the image processing device 11 by executing the operation of the image processing device 11 according to the image processing program.

The program may be stored in a non-transitory computer-readable medium in advance. The non-transitory computer-readable medium is, for example, a flash memory, a magnetic recording device, an optical disc, a magneto-optical recording medium, or a ROM. Distribution of the program is executed by, for example, selling, transferring, or lending a portable medium such as an SD card, a DVD, or a CD-ROM storing the program. "SD" is an abbreviation for secure digital. "DVD" is an abbreviation for digital versatile disc. "CD-ROM" is an abbreviation for compact disc read only memory. The program may be distributed by being stored in a storage of a server in advance and transferred from the server to another computer. The program may be provided as a program product.

For example, the computer temporarily stores, in the main storage device, the program stored in the portable medium or the program transferred from the server. Then, the computer reads, by the processor, the program stored in the main storage device, and executes, by the processor, processing according to the read program. The computer may read the program directly from the portable medium and execute the processing according to the program. Each time the program is transferred from the server to the computer, the computer may sequentially execute processing according to the received program. The processing may be executed by what is called an ASP type service in which the function is implemented only by execution instruction and result acquisition without transferring the program from the server to the computer. "ASP" is an abbreviation for application service provider. The program includes information provided for processing by an electronic computer and conforming to the program. For example, data that is not a direct command to the computer but has a property that defines the processing of the computer corresponds to the "information conforming to the program".

The functions of the image processing device 11 may be partially or entirely implemented by the programmable circuit or the dedicated circuit corresponding to the control unit 41. That is, the functions of the image processing device 11 may be partially or entirely implemented by hardware.

The operation of the image processing system 10 according to the present embodiment will be described with reference to Figs. 8 and 9. The operation of the image processing system 10 corresponds to an image display method according to the present embodiment.

Before a start of the procedure of Fig. 8, the probe 20 is primed by the user. Thereafter, the probe 20 is fitted into the probe connection portion 34 and the probe clamp unit 37 of the drive unit 13, and is connected and fixed to the drive unit 13. Then, the probe 20 is inserted to a target site in the biological tissue 60 such as the blood vessel or the heart.

In step S101, the scan switch included in the switch group 39 is pressed, and what is called a pullback operation is executed by pressing the pullback switch included in the switch group 39. The probe 20 transmits an ultrasound wave inside the biological tissue 60 by the ultrasound transducer 25 that moves backward in the axial direction by the pullback operation. The ultrasound transducer 25 radially transmits the ultrasound wave while moving inside the biological tissue 60. The ultrasound transducer 25 receives a reflected wave of the transmitted ultrasound wave. The probe 20 inputs a signal of the reflected wave received by the ultrasound transducer 25 to the image processing device 11. The control unit 41 of the image processing device 11 processes the input signal to sequentially generate cross-sectional images of the biological tissue 60, thereby acquiring the tomographic data 51, which includes a plurality of cross-sectional images.

Specifically, the probe 20 transmits the ultrasound wave in a plurality of directions from the rotation center to the outside by the ultrasound transducer 25 while rotating the ultrasound transducer 25 in a circumferential direction and moving it in the axial direction inside the biological tissue 60. The ultrasound transducer 25 of the probe 20 receives the reflected wave from a reflecting object present in each of the plurality of directions inside the biological tissue 60. The probe 20 transmits the signal of the received reflected wave to the image processing device 11 via the drive unit 13 and the cable 12. The communication unit 43 of the image processing device 11 receives the signal transmitted from the probe 20. The communication unit 43 executes A/D conversion for the received signal. The communication unit 43 inputs the A/D-converted signal to the control unit 41. The control unit 41 processes the input signal to calculate an intensity value distribution of the reflected wave from the reflecting object present in a transmission direction of the ultrasound wave of the ultrasound transducer 25. The control unit 41 sequentially generates two-dimensional images having a luminance value distribution corresponding to the calculated intensity value distribution as the cross-sectional images of the biological tissue 60, thereby acquiring the tomographic data 51 which is a data set of the cross-sectional images. The control unit 41 stores the acquired tomographic data 51 in the storage unit 42.

In the present embodiment, the signal of the reflected wave received by the ultrasound transducer 25 corresponds to raw data of the tomographic data 51, and the cross-sectional images generated by processing the signal of the reflected wave by the image processing device 11 correspond to processed data of the tomographic data 51.

In a modification of the present embodiment, the control unit 41 of the image processing device 11 may store the signal input from the probe 20 as it is in the storage unit 42 as the tomographic data 51. Alternatively, the control unit 41 may store data indicating the intensity value distribution of the reflected wave calculated by processing the signal input from the probe 20 in the storage unit 42 as the tomographic data 51. That is, the tomographic data 51 is not limited to the data set of the cross-sectional images of the biological tissue 60, and may be data representing a cross section of the biological tissue 60 at each moving position of the ultrasound transducer 25 in any format.

In a modification of the present embodiment, an ultrasound transducer that transmits the ultrasound wave in the plurality of directions without rotating may be used instead of the ultrasound transducer 25 that transmits the ultrasound wave in the plurality of directions while rotating in the circumferential direction.

In a modification of the present embodiment, the tomographic data 51 may be acquired using OFDI or OCT instead of being acquired using IVUS. "OFDI" is an abbreviation for optical frequency domain imaging. "OCT" is an abbreviation for optical coherence tomography. In a case where OFDI or OCT is used, as a sensor that acquires the tomographic data 51 while moving in the lumen 63 of the biological tissue 60, a sensor that acquires the tomographic data 51 by emitting light in the lumen 63 of the biological tissue 60 is used instead of the ultrasound transducer 25 that acquires the tomographic data 51 by transmitting the ultrasound wave in the lumen 63 of the biological tissue 60.

In a modification of the present embodiment, instead of the image processing device 11 generating the data set of the cross-sectional images of the biological tissue 60, another device may generate a similar data set, and the image processing device 11 may acquire the data set from the other device. That is, instead of the control unit 41 of the image processing device 11 processing the IVUS signal to generate the cross-sectional images of the biological tissue 60, another device may process the IVUS signal to generate the cross-sectional images of the biological tissue 60 and input the generated cross-sectional images to the image processing device 11.

In step S102, the control unit 41 of the image processing device 11 generates the three-dimensional data 52 of the biological tissue 60 based on the tomographic data 51 acquired in step S101. That is, the control unit 41 generates the three-dimensional data 52 based on the tomographic data 51 acquired by the sensor. Note that at this time, if already generated three-dimensional data 52 is present, it is preferable to update only data at a location corresponding to the updated tomographic data 51, instead of regenerating all pieces of the three-dimensional data 52 from the beginning. Accordingly, the data processing amount in generating the three-dimensional data 52 can be reduced, and the real-time property of the three-dimensional image 53 in the subsequent step S103 can be improved.

Specifically, the control unit 41 of the image processing device 11 generates the three-dimensional data 52 of the biological tissue 60 by stacking the cross-sectional images of the biological tissue 60 included in the tomographic data 51 stored in the storage unit 42, and converting the same into three-dimensional data. As a method of three-dimensional conversion, any method among a rendering method such as surface rendering or volume rendering, and various types of processing such as texture mapping including environment mapping, and bump mapping, which are associated with the rendering method, can be used. The control unit 41 stores the generated three-dimensional data 52 in the storage unit 42.

When the elongated medical instrument 67 such as a catheter different from the IVUS catheter or the like is inserted into the biological tissue 60, the tomographic data 51 includes data of the elongated medical instrument 67, similarly to the data of the biological tissue 60. Therefore, in step S102, the three-dimensional data 52 generated by the control unit 41 also includes the data of the elongated medical instrument 67 similarly to the data of the biological tissue 60.

The control unit 41 of the image processing device 11 classifies pixel groups of the cross-sectional images included in the tomographic data 51 acquired in step S101 into two or more classes. These two or more classes include at least a class of "tissue" to which the biological tissue 60 belongs and a class of "medical instrument" to which the elongated medical instrument 67 belongs, and may further include a class of "blood cell", a class of "indwelling object" of an indwelling stent or the like, or a class of "lesion" of lime, plaque, or the like. As a classification method, any method may be used, but in the present embodiment, a method of classifying the pixel groups of the cross-sectional images by a trained model is used. The trained model is trained by performing machine learning in advance so that the trained model can detect a region corresponding to each class from a cross-sectional image of IVUS as a sample.

In step S103, the control unit 41 of the image processing device 11 causes the display 16 to display the three-dimensional data 52 generated in step S102 as the three-dimensional image 53. At this time, the control unit 41 may set an angle for displaying the three-dimensional image 53 to any angle. The control unit 41 causes the display 16 to display the latest cross-sectional image included in the tomographic data 51 acquired in step S101 together with the three-dimensional image 53.

Specifically, the control unit 41 of the image processing device 11 generates the three-dimensional image 53 based on the three-dimensional data 52 stored in the storage unit 42. The three-dimensional image 53 includes a three-dimensional object group including a three-dimensional object representing the biological tissue 60 and a three-dimensional object representing the elongated medical instrument 67. That is, the control unit 41 generates a three-dimensional object of the biological tissue 60 from the data of the biological tissue 60 stored in the storage unit 42, and generates a three-dimensional object of the elongated medical instrument 67 from the data of the elongated medical instrument 67 stored in the storage unit 42. The control unit 41 causes the display 16 to display the latest cross-sectional image among the cross-sectional images of the biological tissue 60 included in the tomographic data 51 stored in the storage unit 42 and the generated three-dimensional image 53 via the output unit 45.

In the present embodiment, the control unit 41 of the image processing device 11 specifies, as a contact point Pi, a point of the biological tissue 60 in contact with the distal end of the elongated medical instrument 67 in the three-dimensional data 52. Then, in the three-dimensional image 53, the control unit 41 sets the color of the voxels corresponding to the contact point Pi to a predetermined color. The "predetermined color" is red in the present embodiment, but may be any color as long as the voxels corresponding to the contact point Pi can be distinguished from other voxel groups.

In the examples of Figs. 18 and 19, the control unit 41 of the image processing device 11 specifies, as a contact spot 68, a certain range of the biological tissue 60 including the contact point Pi as the center in the three-dimensional data 52. Then, in the three-dimensional image 53, the control unit 41 sets the color of the voxel group corresponding to the contact spot 68 to a predetermined color.

In the example of Fig. 18, the center of the contact spot 68 is viewed straight from the viewpoint, but it is assumed that the center of the contact spot 68 is shifted to either the right or the left as viewed from the viewpoint. In that case, the cross section of the contact spot 68, that is, the cross section of the distal end of the septal puncture needle cannot be seen only by rotating the viewpoint in the circumferential direction by 90 degrees as described later. Therefore, the control unit 41 of the image processing device 11 receives, via the input unit 44, an operation of adjusting the viewpoint and moving the center of the contact spot 68 to the center in the left-right direction as viewed from the viewpoint. By moving the center of the contact spot 68 to the center in the left-right direction as viewed from the viewpoint and then rotating the viewpoint by 90 degrees in the circumferential direction, the position of the cutting region 62 can be adjusted such that the center of the contact spot 68 is located on the cutting plane. As a result, as in the example of Fig. 19, the center of the contact spot 68 can be viewed exactly from the side, and the structure of the left atrium ahead of the distal end of the septal puncture needle can be confirmed exactly from the side.

The contact point Pi may be specified by any process, but in the present embodiment, the contact point Pi is specified by the following process.

The control unit 41 of the image processing device 11 analyzes the tomographic data 51 stored in the storage unit 42 and detects the position of the biological tissue 60 being in contact with the distal end of the elongated medical instrument 67. As a method of analyzing the tomographic data 51, any method may be used, but the present embodiment uses a method of detecting the biological tissue 60 and the distal end of the elongated medical instrument 67 in the cross-sectional image included in the tomographic data 51 and measuring the distance between the biological tissue 60 and the distal end of the elongated medical instrument 67 to determine whether or not the biological tissue 60 and the distal end of the elongated medical instrument 67 are in contact with each other. The control unit 41 specifies a point corresponding to the detected position in the three-dimensional data 52 as the contact point Pi.

In a modification of the present embodiment, the control unit 41 of the image processing device 11 may analyze the three-dimensional data 52 to specify the contact point Pi. As a method of analyzing the three-dimensional data 52, any method may be used. For example, a method of detecting the distal end of the elongated medical instrument 67 included in the three-dimensional data 52 and measuring the distance between the biological tissue 60 and the distal end of the elongated medical instrument 67 to determine whether or not the biological tissue 60 and the distal end of the elongated medical instrument 67 are in contact with each other.

In this modification, the control unit 41 of the image processing device 11 may receive input of positional data indicating the position of the biological tissue 60 being in contact with the distal end of the elongated medical instrument 67. Specifically, the control unit 41 may receive the input of the positional data from an external system that determines whether or not the distal end of the elongated medical instrument 67 is in contact with the inner wall of the biological tissue 60 using a sensor such as an electrode disposed at the distal end of the elongated medical instrument 67 via the communication unit 43 or the input unit 44. Then, the control unit 41 may correct the analysis result of the three-dimensional data 52 with reference to the input positional data.

In a modification of the present embodiment, the control unit 41 may specify a point corresponding to the position indicated by the positional data input from the external system as described above in the three-dimensional data 52 as the contact point Pi without analyzing the three-dimensional data 52.

In step S104, if there is an operation of setting the angle for displaying the three-dimensional image 53 as a change operation by the user, processing of step S105 is executed. If there is no change operation by the user, processing of step S106 is executed.

In step S105, the control unit 41 of the image processing device 11 receives, via the input unit 44, the operation of setting the angle for displaying the three-dimensional image 53. The control unit 41 adjusts the angle for displaying the three-dimensional image 53 to the set angle. In step S103, the control unit 41 causes the display 16 to display the three-dimensional image 53 at the angle set in step S105.

Specifically, the control unit 41 of the image processing device 11 receives, via the input unit 44, an operation by the user of rotating the three-dimensional image 53 displayed on the display 16 by using the keyboard 14, the mouse 15, or the touch screen disposed integrally with the display 16. The control unit 41 interactively adjusts the angle for displaying the three-dimensional image 53 on the display 16 according to the operation by the user. Alternatively, the control unit 41 receives, via the input unit 44, an operation by the user of inputting a numerical value of the angle for displaying the three-dimensional image 53 by using the keyboard 14, the mouse 15, or the touch screen disposed integrally with the display 16. The control unit 41 adjusts the angle for displaying the three-dimensional image 53 on the display 16 in accordance with the input numerical value.

In step S106, if the tomographic data 51 is updated, processing of steps S107 and S108 is executed. If the tomographic data 51 is not updated, the presence or absence of the change operation by the user is confirmed again in step S104.

In step S107, similarly to the processing in step S101, the control unit 41 of the image processing device 11 processes the signal input from the probe 20 to newly generate cross-sectional images of the biological tissue 60, thereby acquiring the tomographic data 51 including at least one new cross-sectional image.

In step S108, the control unit 41 of the image processing device 11 updates the three-dimensional data 52 of the biological tissue 60 based on the tomographic data 51 acquired in step S107. That is, the control unit 41 updates the three-dimensional data 52 based on the tomographic data 51 acquired by the sensor. Then, in step S103, the control unit 41 causes the display 16 to display the three-dimensional data 52 updated in step S108 as the three-dimensional image 53. The control unit 41 causes the display 16 to display the latest cross-sectional image included in the tomographic data 51 acquired in step S107 together with the three-dimensional image 53. In step S108, it is preferable to update only data at a location corresponding to the updated tomographic data 51. Accordingly, the data processing amount in generating the three-dimensional data 52 can be reduced, and the real-time property of the three-dimensional image 53 can be improved in step S108.

In step S111, if there is an operation of setting the cutting region 62 as a setting operation by the user, the processing of step S112 is executed.

In step S112, the control unit 41 of the image processing device 11 receives, via the input unit 44, the operation of setting the cutting region 62.

Specifically, the control unit 41 of the image processing device 11 receives an operation of setting the region 65 corresponding to the cutting region 62 with respect to the cross-sectional image displayed on the display 16 in step S103 via the input unit 44. In the present embodiment, the control unit 41 receives an operation of setting the two straight lines L1 and L2 extending from one point M in the cross-sectional image as the operation of setting the region 65 corresponding to the cutting region 62.

More specifically, the control unit 41 of the image processing device 11 receives, via the input unit 44, an operation by the user of designating the base angle and the opening angle on the operation panel 81 as illustrated in Figs. 18 and 19 using the keyboard 14, the mouse 15, or the touch screen disposed integrally with the display 16. That is, as the operation of setting the two straight lines L1 and L2, the control unit 41 receives an operation of designating the direction of the straight line L1 of the two straight lines L1 and L2 and the angle formed by the two straight lines L1 and L2. Here, it is assumed that the check box 85 of the operation panel 81 is in a checked state, that is, use of a centroid is selected.

In a modification of the present embodiment, the control unit 41 of the image processing device 11 may receive, via the input unit 44, an operation by the user of drawing the two straight lines L1 and L2 on a cross-sectional image displayed on the display 16 by using the keyboard 14, the mouse 15, or the touch screen disposed integrally with the display 16. That is, the control unit 41 may receive an operation of drawing the two straight lines L1 and L2 on the cross-sectional image as the operation of setting the two straight lines L1 and L2.

In step S113, the control unit 41 of the image processing device 11 calculates the centroid positions of the plurality of lateral cross sections of the lumen 63 of the biological tissue 60 by using the latest three-dimensional data 52 stored in the storage unit 42. The latest three-dimensional data 52 is the three-dimensional data 52 generated in step S102 if the processing in step S108 is not executed, and is the three-dimensional data 52 updated in step S108 if the processing in step S108 is executed. Note that at this time, if already generated three-dimensional data 52 is present, it is preferable to update only data at a location corresponding to the updated tomographic data 51, instead of regenerating all of the three-dimensional data 52 from the beginning. Accordingly, the data processing amount in generating the three-dimensional data 52 can be reduced, and the real-time property of the three-dimensional image 53 in the subsequent step S117 can be improved.

Specifically, as illustrated in Fig. 10, if the control unit 41 of the image processing device 11 generates a corresponding new cross-sectional image in step S107 for each of the plurality of cross-sectional images generated in step S101, the control unit 41 replaces each of the plurality of cross-sectional images generated in step S101 with the new cross-sectional image, and then binarizes the cross-sectional image. As illustrated in Fig. 11, the control unit 41 extracts a point cloud on the inner surface of the biological tissue 60 from the binarized cross-sectional image. For example, the control unit 41 extracts a point cloud on an inner surface of a blood vessel by extracting points corresponding to an inner surface of a main blood vessel one by one along a longitudinal direction of the cross-sectional image with an r-axis as a horizontal axis and a $\theta$-axis as a vertical axis. The control unit 41 may simply obtain the centroid of the extracted point cloud on the inner surface, but in that case, since the point cloud is not uniformly sampled over the inner surface, a centroid position shifts. Therefore, in the present embodiment, the control unit 41 calculates the convex hull of the extracted point cloud on the inner surface, and calculates a centroid position $C_n = (C_x, C_y)$ by using a formula for obtaining the centroid of a polygon as follows. However, in the following formula, it is assumed that n vertices $(x_0, y_0)$,

$(x_1, y_1), ..., (x_{n-1}, y_{n-1})$ are present on the convex hull counterclockwise as the point cloud on the inner surface as illustrated in Fig. 11, and $(x_n, y_n)$ is regarded as $(x_0, y_0)$.

[Math. 2]

$$C_x = \frac{1}{6A} \sum_{i=0}^{n-1} (x_i + x_{i+1})(x_i y_{i+1} - x_{i+1} y_i)$$

$$C_y = \frac{1}{6A} \sum_{i=0}^{n-1} (y_i + y_{i+1})(x_i y_{i+1} - x_{i+1} y_i)$$

$$A = \frac{1}{2} \sum_{i=0}^{n-1} (x_i y_{i+1} - x_{i+1} y_i)$$

The centroid positions obtained as results are illustrated in Fig. 12. In Fig. 12, a point Cn is the center of the cross-sectional image. A point Bp is a centroid of the point cloud on the inner surface. A point Bv is a centroid of the vertices of the polygon. A point Bx is a centroid of the polygon serving as a convex hull.

As a method of calculating the centroid position of the blood vessel, a method other than the method of calculating the centroid position of the polygon serving as the convex hull may be used. For example, with respect to an original cross-sectional image that is not binarized, a method of calculating a center position of the maximum circle that falls within the main blood vessel as the centroid position may be used. Alternatively, with respect to the binarized cross-sectional image having the r-axis as the horizontal axis and the θ-axis as the vertical axis, a method of calculating an average position of pixels in a main blood vessel region as the centroid position may be used. The same method as described above may also be used when the biological tissue 60 is not a blood vessel.

In step S114, the control unit 41 of the image processing device 11 smooths calculation results of the centroid positions in step S113.

As illustrated in Fig. 13, when the calculation results of the centroid positions are viewed as a time function, it can be seen that an influence of pulsation is large. Therefore, in the present embodiment, the control unit 41 of the image processing device 11 smooths the calculation results of the centroid positions by using moving averages as indicated by a broken line in Fig. 14.

As a smoothing method, a method other than the movement average may be used. For example, exponential smoothing method, kernel method, local regression, Ramer-Douglas-Peucker algorithm, Savitzky-Golay method, smoothed spline, or SGM may be used. Alternatively, a method of executing the fast Fourier transform and then removing a highfrequency component may be used. Alternatively, Kalman filter or a low-pass filter such as Butterworth filter, Chebyshev filter, digital filter, elliptical filter, or KZ filter may be used. "SGM" is an abbreviation for stretched grid method. "KZ" is an abbreviation for Kolmogorov-Zurbenko.

Simple smoothing may cause the centroid positions to enter the tissue. In this case, the control unit 41 may divide the calculation results of the centroid positions, in the longitudinal direction of the lumen 63 of the biological tissue 60, according to positions of the plurality of lateral cross sections of the lumen 63 of the biological tissue 60, and may smooth each of the divided calculation results. That is, when a curve of the centroid positions as indicated by the broken line in Fig. 14 overlaps a tissue region, the control unit 41 may divide the curve of the centroid positions into a plurality of sections and execute individual smoothing for each section. Alternatively, the control unit 41 may adjust a degree of smoothing to be executed on the calculation results of the centroid positions according to the positions of the plurality of lateral cross sections of the lumen 63 of the biological tissue 60 in the longitudinal direction of the lumen 63 of the biological tissue 60. That is, when the curve of the centroid positions as indicated by the broken line in Fig. 14 overlaps the tissue region, the control unit 41 may decrease the degree of smoothing to be executed for a part of the sections including the overlapping points.

In step S115, as illustrated in Fig. 4, the control unit 41 of the image processing device 11 sets two planes intersecting at the single line Lb passing through the centroid positions calculated in step S113, as cutting planes P1 and P2. In the present embodiment, the control unit 41 smooths the calculation results of the centroid positions in step S114, and then sets the cutting planes P1 and P2, but the processing of step S114 may be omitted.

Specifically, the control unit 41 of the image processing device 11 sets a curve of the centroid positions obtained as a result of the smoothing in step S114 as the line Lb. The control unit 41 sets two planes intersecting at the set line Lb and including, respectively, the two straight lines L1 and L2 set in step S112 as the cutting planes P1 and P2. The control unit 41 identifies three-dimensional coordinates intersecting with the cutting planes P1 and P2 of the biological tissue 60 in the latest three-dimensional data 52 stored in the storage unit 42 as the three-dimensional coordinates of the edges of the opening exposing the lumen 63 of the biological tissue 60 in the three-dimensional image 53. The control unit 41 stores the identified three-dimensional coordinates in the storage unit 42.

In step S116, the control unit 41 of the image processing device 11 forms, in the three-dimensional data 52, a region interposed between the cutting planes P1 and P2 and exposing the lumen 63 of the biological tissue 60 in the three-dimensional image 53, as a cutting region 62.

Specifically, the control unit 41 of the image processing device 11 sets a portion identified by the three-dimensional coordinates stored in the storage unit 42 in the latest three-dimensional data 52 stored in the storage unit 42 to be hidden or transparent when the three-dimensional image 53 is displayed on the display 16. That is, the control unit 41 forms the cutting region 62 in accordance with the region 65 set in step S112.

In step S117, the control unit 41 of the image processing device 11 causes the display 16 to display the three-dimensional data 52 in which the cutting region 62 is formed in step S116 as the three-dimensional image 53. The control unit 41 causes the display 16 to display the two-dimensional image 56 representing the cross section 64 indicated by the tomographic data 51 newly acquired by the sensor and represented by the cross-sectional image displayed on the display 16 in step S103 and the region 65 corresponding to the cutting region 62 in the cross section 64 together with the three-dimensional image 53.

Specifically, the control unit 41 of the image processing device 11 generates the two-dimensional image 56 as illustrated in Figs. 18 and 19 by processing the latest cross-sectional image among the cross-sectional images of the biological tissue 60 included in the tomographic data 51 stored in the storage unit 42. The control unit 41 generates the three-dimensional image 53 as illustrated in Figs. 18 and 19 in which a portion identified by the three-dimensional coordinates stored in the storage unit 42 is hidden or transparent. The control unit 41 causes the display 16 to display the generated two-dimensional image 56 and three-dimensional image 53 via the output unit 45.

In the present embodiment, as illustrated in Figs. 18 and 19, the control unit 41 of the image processing device 11 generates, as the two-dimensional image 56, an image showing the region 65 corresponding to the cutting region 62 in a color different from that of the remaining region. For example, it is conceivable to change a white portion in a typical IVUS image to red in the region 65.

In step S118, if there is an operation of setting the cutting region 62 as a change operation by the user, the processing of step S119 is executed. If there is no change operation by the user, processing of step S120 is executed.

In step S119, the control unit 41 of the image processing device 11 receives, via the input unit 44, the operation of setting the cutting region 62, similarly to the processing in step S112. Then, the processing in and after step S115 is executed.

If the tomographic data 51 is updated in step S120, the processing in steps S121 and S122 is executed. If the tomographic data 51 is not updated, the presence or absence of the change operation by the user is confirmed again in step S118.

In step S121, similarly to the processing in step S101 or step S107, the control unit 41 of the image processing device 11 processes the signal input from the probe 20 to newly generate cross-sectional images of the biological tissue 60, thereby acquiring the tomographic data 51 including at least one new cross-sectional image.

In step S122, the control unit 41 of the image processing device 11 updates the three-dimensional data 52 of the biological tissue 60 based on the tomographic data 51 acquired in step S121. Thereafter, the processing in and after step S113 is executed. In step S122, it is preferable to update only data at a location corresponding to the updated tomographic data 51. Accordingly, the data processing amount in generating the three-dimensional data 52 can be reduced, and the real-time property of data processing in and after step S113 can be improved.

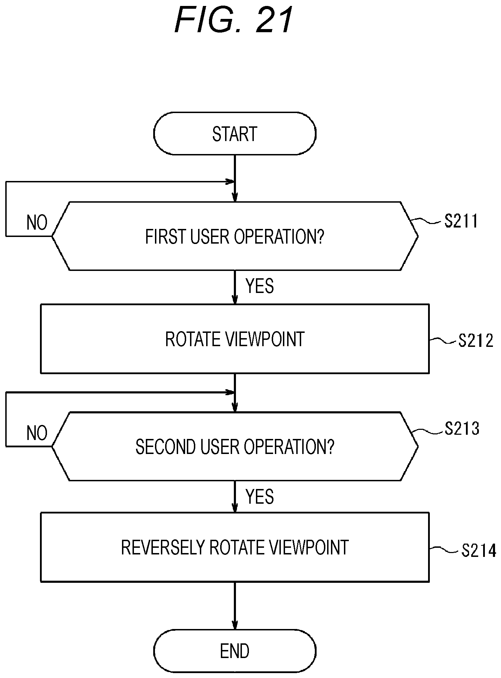

The operation of the image processing system 10 according to the present embodiment will be further described with reference to Fig. 21.

In step S211, when an operation of pressing the button 89 displayed on the display 16 is performed as a user operation of requesting rotation of the viewpoint for displaying the three-dimensional image 53 on the display 16, the control unit 41 of the image processing device 11 receives the operation via the input unit 44.

In step S212, the control unit 41 of the image processing device 11 rotates the viewpoint in accordance with the user operation performed in step S211. Specifically, the control unit 41 changes the position of the cutting region 62 from the first position at the time of the user operation in step S211 to the second position after rotation around the rotation axis passing through the reference point located in the lumen 63 on the reference plane and extending in the direction perpendicular to the reference plane, the reference plane extending in the horizontal direction in the three-dimensional image 53 and including the viewpoint. Then, the control unit 41 rotates the viewpoint around the rotation axis according to the second position. In the present embodiment, the control unit 41 rotates the viewpoint by 90 degrees around the rotation axis. In the examples of Figs. 18 and 19, the control unit 41 switches the three-dimensional image 53 from an image in which the oval fossa 66 is captured from the front as illustrated in Fig. 18 to an image in which the oval fossa 66 is captured from the side as illustrated in Fig. 19. The control unit 41 switches the notation of the button 89 from "+90" and a triangle pointing to the right as illustrated in Fig. 18 to "-90" and a triangle pointing to the left as illustrated in Fig. 19. Although not essential, the control unit 41 may set the second position such that, when the position of the cutting region 62 is the second position, a part of the contact spot 68 is located on the cutting plane formed by the cutting region 62. That is, the control unit 41 may adjust the position of the cutting region 62 such that the contact spot 68 is located on the cutting plane, that is, such that the contact spot 68 is easily visible. This position adjustment may be performed only when there is an explicit request from the user. Alternatively, the control unit 41 may change the position of the cutting region 62 to the second position after adjusting the viewpoint to position the viewpoint on the plane including the contact point Pi and the rotation axis. This viewpoint adjustment may be performed only when there is an explicit request from the user. Alternatively, the control unit 41 may set the reference point such that the reference point is located on a projection line obtained by projecting a straight line connecting the contact point Pi and the viewpoint onto the reference plane. That is, the control unit 41 may adjust the rotation axis such that the contact point Pi, the viewpoint, and one point on the rotation axis are aligned. This rotation axis adjustment may be performed only when there is an explicit request from the user.

In step S213, when an operation of pressing the button 89 displayed on the display 16 is performed again as a user operation of requesting rotation of the viewpoint, the control unit 41 of the image processing device 11 receives the operation via the input unit 44.

In step S214, the control unit 41 of the image processing device 11 reversely rotates the viewpoint according to the user operation performed in step S213. Specifically, the control unit 41 changes the position of the cutting region 62 from the second position to the first position around the rotation axis. Then, the control unit 41 reversely rotates the viewpoint around the rotation axis according to the first position. In the present embodiment, the control unit 41 reversely rotates the viewpoint by 90 degrees around the rotation axis. In the examples of Figs. 18 and 19, the control unit 41 switches the three-dimensional image 53 from an image in which the oval fossa 66 is captured from the side as illustrated in Fig. 19 to an image in which the oval fossa 66 is captured from the front as illustrated in Fig. 18. The control unit 41 switches the notation of the button 89 from "-90" and a triangle pointing to the left as illustrated in Fig. 19 to "+90" and a triangle pointing to the right as illustrated in Fig. 18.

The procedure of Fig. 21 may be repeated any number of times.

For example, the image processing device, the image processing system, the image display method, and the image processing program according to the present embodiment correspond to an image processing device, an image processing system, an image display method, and an image processing program according to the following appendices, respectively.

Appendix 1.

An image processing device that causes a display to display three-dimensional data representing a biological tissue as a three-dimensional image, forms, in the three-dimensional data, a cutting region exposing a lumen of the biological tissue in the three-dimensional image, and adjusts a viewpoint for displaying the three-dimensional image on the display according to a position of the cutting region, the image processing device including
a control unit that changes, upon receiving a user operation of requesting rotation of the viewpoint, the position of the cutting region from a first position at time of the user operation to a second position after rotation around a rotation axis passing through a reference point located in the lumen on a reference plane and extending in a direction perpendicular to the reference plane, the reference plane extending in a horizontal direction in the three-dimensional image and including the viewpoint, and rotates the viewpoint around the rotation axis according to the second position.

Appendix 2.

The image processing device according to Appendix 1, in which the reference point is a centroid of the lumen on the reference plane.

Appendix 3.

The image processing device according to Appendix 1 or 2, in which the control unit rotates the viewpoint by 90 degrees around the rotation axis upon receiving the user operation.

Appendix 4.

The image processing device according to any one of Appendices 1 to 3, in which the control unit receives, as the user operation, an operation of pressing a button displayed on the display.

Appendix 5.

The image processing device according to any one of Appendices 1 to 4, in which the control unit causes the display to display a three-dimensional object representing an elongated medical instrument inserted into the lumen so as to be included in the three-dimensional image.

Appendix 6.

The image processing device according to Appendix 5, in which the control unit adjusts the viewpoint to position the viewpoint on a plane including a contact point of the biological tissue being in contact with a distal end of the elongated medical instrument and the rotation axis, and changes a position of the cutting region to the second position.

Appendix 7.

The image processing device according to Appendix 5, in which the reference point is located on a projection line obtained by projecting, on the reference plane, a straight line connecting the viewpoint and a contact point of the biological tissue being in contact with the distal end of the elongated medical instrument.

Appendix 8.

The image processing device according to Appendix 5, in which, when the position of the cutting region is the second position, a part of a contact spot of the biological tissue is located on a cutting plane formed by the cutting region, the contact spot being a certain range including, as a center, the contact point being in contact with the distal end of the elongated medical instrument.

Appendix 9.

The image processing device according to any one of Appendices 1 to 8, in which the control unit further causes the display to display a cross-sectional image of the biological tissue disposed on a screen same as the three-dimensional image.

Appendix 10.

An image processing system including:

the image processing device according to any one of Appendices 1 to 9; and
the display.

Appendix 11.

An image display method of causing a display to display three-dimensional data representing a biological tissue as a three-dimensional image, forming, in the three-dimensional data, a cutting region exposing a lumen of the biological tissue in the three-dimensional image, and adjusting a viewpoint for displaying the three-dimensional image on the display according to a position of the cutting region, the image display method including:

receiving a user operation of requesting rotation of the viewpoint;
changing the position of the cutting region from a first position at time of the user operation to a second position after rotation around a rotation axis passing through a reference point located in the lumen on a reference plane and extending in a direction perpendicular to the reference plane, the reference plane extending in a horizontal direction in the three-dimensional image and including the viewpoint; and
rotating the viewpoint around the rotation axis according to the second position.

Appendix 12.

An image processing program that causes a computer to execute processing, the computer causing a display to display three-dimensional data representing a biological tissue as a three-dimensional image, forming, in the three-dimensional data, a cutting region exposing a lumen of the biological tissue in the three-dimensional image, and adjusting a viewpoint for displaying the three-dimensional image on the display according to a position of the cutting region, the processing including

changing, upon reception of a user operation of requesting rotation of the viewpoint, the position of the cutting region from a first position at time of the user operation to a second position after rotation around a rotation axis passing through a reference point located in the lumen on a reference plane and extending in a direction perpendicular to the reference plane, the reference plane extending in a horizontal direction in the three-dimensional image and including the viewpoint, and rotating the viewpoint around the rotation axis according to the second position.

The present disclosure is not limited to the above-described embodiment. For example, two or more blocks illustrated in the block diagram may be integrated, or one block may be divided. Instead of executing two or more steps described in the flowchart in time series according to the description, the steps may be executed in parallel or in a different order according to the processing capability of the device that executes each step or as necessary. In addition, modifications can be made without departing from the gist of the present disclosure.

Reference Signs List

|     |                             |
| --- | --------------------------- |
| 10  | Image processing system     |
| 11  | Image processing device     |
| 12  | Cable                       |
| 13  | Drive unit                  |
| 14  | Keyboard                    |
| 15  | Mouse                       |
| 16  | Display                     |
| 17  | Connection terminal         |
| 18  | Cart unit                   |
| 20  | Probe                       |
| 21  | Drive shaft                 |
| 22  | Hub                         |
| 23  | Sheath                      |
| 24  | Outer tube                  |
| 25  | Ultrasound transducer       |
| 26  | Relay connector             |
| 31  | Scanner unit                |
| 32  | Slide unit                  |
| 33  | Bottom cover                |
| 34  | Probe connection portion    |
| 35  | Scanner motor               |
| 36  | Insertion port              |
| 37  | Probe clamp unit            |
| 38  | Slide motor                 |
| 39  | Switch group                |
| 41  | Control unit                |
| 42  | Storage unit                |
| 43  | Communication unit          |
| 44  | Input unit                  |
| 45  | Output unit                 |
| 46  | Movement control function   |
| 51  | Tomographic data            |
| 52  | Three-dimensional data      |
| 53  | Three-dimensional image     |
| 54a | First voxel group           |
| 54b | Second voxel group          |

| 54c | Third voxel group |
| 54d | Fourth voxel group |
| 55 | Voxel group |
| 56, 56a, 56b | Two-dimensional image |
| 60 | Biological tissue |
| 61 | Inner surface |
| 62 | Cutting region |
| 63 | Lumen |
| 64 | Cross section |
| 65, | 65a, 65b Region |
| 66 | Oval fossa |
| 67 | Elongated medical instrument |
| 68 | Contact spot |
| 71 | Camera |
| 72 | Mark |
| 80 | Screen |
| 81 | Operation panel |
| 82 | Check box |
| 83 | Slider |
| 84 | Slider |
| 85 | Check box |
| 86 | Fifth graphic element |
| 87a | First graphic element |
| 87b | Second graphic element |
| 87c | Third graphic element |
| 87d | Fourth graphic element |
| 88b, 88c, 88d | Movement button |
| 89 | Button |

**Claims**

1. An image processing device configured to cause a display to display, based on tomographic data acquired by a sensor moving in a lumen of a biological tissue, an image representing the biological tissue and display a first element on a screen same as the image, the first element representing a position of the sensor and being displaced as the sensor moves, the image processing device comprising
a control unit configured to cause the display to display, upon receiving a user operation of requesting marking of the position of the sensor, a second element together with the first element, the second element being fixed at a position same as a position of the first element at time of the user operation.

2. The image processing device according to claim 1, wherein the control unit is configured to set a color of the second element to a color different from a color of the first element.

3. The image processing device according to claim 1 or 2, wherein the control unit is configured to move the sensor to a position corresponding to a position of the second element upon receiving an operation of requesting movement of the sensor to the position corresponding to the position of the second element.

4. The image processing device according to any one of claims 1 to 3, wherein the control unit is configured to cause the display to display, upon receiving the user operation again, a third element together with the first element and the second element, the third element being fixed at a position same as a position of the first element at time of the user operation performed again.

5. The image processing device according to claim 4, wherein the control unit is configured to set a color of the third element to a color different from the color of the second element.

6. The image processing device according to claim 4 or 5, wherein the control unit is configured to cause the display to display a fourth element together with the first element, the second element, and the third element, the fourth element being fixed at a position between the second element and the third element.

7. The image processing device according to claim 6, wherein the control unit is configured to calculate an intermediate position between the second element and the third element as the position between the second element and the third element.

8. The image processing device according to claim 6 or 7, wherein the control unit is configured to set a color of the fourth element to a color different from the color of the second element and the color of the third element.

9. The image processing device according to any one of claims 6 to 8, wherein the control unit is configured to move the sensor to a position corresponding to a position of the fourth element upon receiving an operation of requesting movement of the sensor to the position corresponding to the position of the fourth element.

10. The image processing device according to any one of claims 4 to 9, wherein the control unit is configured to set a color of a region between a cross section corresponding to the position of the second element and a cross section corresponding to a position of the third element to a color different from a color of an adjacent region in a three-dimensional image that is the image.

11. The image processing device according to any one of claims 1 to 10, wherein the control unit is configured to combine a graphic element group that is an element group including the first element and the second element and an elongated graphic element representing a movement range of the sensor and to cause the display to display the graphic element group and the elongated graphic element.

12. The image processing device according to claim 11, wherein the control unit is configured to cause the display to display the elongated graphic element in a direction in which a long axis direction of the elongated graphic element is parallel to a longitudinal direction of the lumen in the three-dimensional image that is the image.

13. The image processing device according to claim 1, wherein the control unit is configured to define the first element and the second element in a three-dimensional image that is the image, the first element being defined as at least a voxel representing an inner surface of the biological tissue or a voxel adjacent to the voxel representing the inner surface and representing the lumen in a first voxel group corresponding to a position of the sensor, the second element being defined as at least a voxel representing the inner surface or a voxel adjacent to the voxel representing the inner surface and representing the lumen in a second voxel group corresponding to a position of the sensor at time of the user operation, and colors the second element distinguishably from the first element.

14. The image processing device according to any one of claims 1 to 13, wherein the control unit is configured to receive an operation of pressing one or more predetermined keys as the user operation.

15. An image processing system comprising:

the image processing device according to any one of claims 1 to 14; and
a probe including the sensor.

16. The image processing system according to claim 15 further comprising the display.

17. An image display method of causing a display to display, based on tomographic data acquired by a sensor moving in a lumen of a biological tissue, an image representing the biological tissue and display a first element on a screen same as the image, the first element representing a position of the sensor and being displaced as the sensor moves, the image display method comprising:

receiving a user operation of requesting marking of the position of the sensor; and
causing the display to display a second element together with the first element, the second element being fixed at a position same as a position of the first element at time of the user operation.

18. An image processing program configured to cause a computer to execute processing, the computer causing a display to display, based on tomographic data acquired by a sensor moving in a lumen of a biological tissue, an image representing the biological tissue and display a first element on a screen same as the image, the first element representing a position of the sensor and being displaced as the sensor moves, the processing comprising:
causing the display to display, upon reception of a user operation of requesting marking of the position of the sensor, a second element together with the first element, the second element being fixed at a position same as a position of the first element at time of the user operation.

# FIG. 1

## FIG. 2

SETTING OF CUTTING REGION

☑ ACTIVATE

BASE ANGLE ▬

OPENING ANGLE ▬

USE CENTROID ☑

# FIG. 3

# FIG. 4

# FIG. 5

11

IMAGE PROCESSING DEVICE

CONTROL UNIT ~41

51
TOMOGRAPHIC DATA

52
THREE-DIMENSIONAL DATA

53
THREE-DIMENSIONAL IMAGE

42 STORAGE UNIT

43 COMMUNICATION UNIT

44 INPUT UNIT

45 OUTPUT UNIT

# FIG. 6

EP 4 400 059 A1

SETTING OF CUTTING REGION

☑ ACTIVATE

BASE ANGLE

OPENING ANGLE

USE CENTROID ☑

*FIG. 7*

EP 4 400 059 A1

# FIG. 8

```
                    ┌──────────┐
                    │  START   │
                    └──────────┘
                         │
                         ▼
S101 ┌─────────────────────────────────────────┐
     │        ACQUIRE TOMOGRAPHIC DATA          │
     └─────────────────────────────────────────┘
                         │
                         ▼
S102 ┌─────────────────────────────────────────┐
     │      GENERATE THREE-DIMENSIONAL DATA     │
     └─────────────────────────────────────────┘
                         │
                         ▼
S103 ┌─────────────────────────────────────────┐
     │   DISPLAY LATEST CROSS-SECTIONAL IMAGE   │
     │       AND THREE-DIMENSIONAL IMAGE        │
     └─────────────────────────────────────────┘
                         │
                         ▼
S104  ◁─────────────────────────────────────────▷  NO
         CHANGE OPERATION BY USER?
                         │ YES
                         ▼
S105 ┌─────────────────────────────────────────┐
     │          RECEIVE CHANGE OPERATION        │
     └─────────────────────────────────────────┘

S106  ◁─────────────────────────────────────────▷  NO
         TOMOGRAPHIC DATA UPDATED?
                         │ YES
                         ▼
S107 ┌─────────────────────────────────────────┐
     │        ACQUIRE TOMOGRAPHIC DATA          │
     └─────────────────────────────────────────┘
                         │
                         ▼
S108 ┌─────────────────────────────────────────┐
     │      UPDATE THREE-DIMENSIONAL DATA       │
     └─────────────────────────────────────────┘
```

## FIG. 9

```
                    ┌─────────────┐
                    │    START    │
                    └──────┬──────┘
                           │ ◄─────────────────────────┐
  S111                     ▼                           │
  ⟨  SETTING OPERATION BY USER?              ⟩── NO ───┘
                           │
                          YES
                           ▼
  S112   ┌──────────────────────────────────────────┐
         │         RECEIVE SETTING OPERATION         │
         └──────────────────┬───────────────────────┘
                            │ ◄──────────────────────────────┐
                            ▼                                 │
  S113   ┌──────────────────────────────────────────┐        │
         │    CALCULATE CENTROID POSITIONS OF        │        │
         │         MULTIPLE CROSS SECTIONS           │        │
         └──────────────────┬───────────────────────┘        │
                            ▼                                 │
  S114   ┌──────────────────────────────────────────┐        │
         │            EXECUTE SMOOTHING              │        │
         └──────────────────┬───────────────────────┘        │
                            ▼ ◄──────────┐                    │
  S115   ┌──────────────────────────────┴───────────┐        │
         │         SET PAIR OF CUTTING PLANES        │        │
         └──────────────────┬───────────────────────┘        │
                            ▼                                 │
  S116   ┌──────────────────────────────────────────┐        │
         │  FORM CUTTING REGION IN THREE-DIMENSIONAL DATA│    │
         └──────────────────┬───────────────────────┘        │
                            ▼                                 │
  S117   ┌──────────────────────────────────────────┐        │
         │       DISPLAY TWO-DIMENSIONAL IMAGE       │        │
         │       AND THREE-DIMENSIONAL IMAGE         │        │
         └──────────────────┬───────────────────────┘        │
  S118                      │ ◄───────────────────┐           │
  ⟨       CHANGE OPERATION BY USER?           ⟩── NO ─┐       │
                            │                         │       │
                           YES                        │       │
                            ▼                         │       │
  S119   ┌──────────────────────────────────────────┐ │       │
         │         RECEIVE CHANGE OPERATION          │ │       │
         └──────────────────┬───────────────────────┘ │       │
                            │ ◄───────────────────────┘       │
  S120                      ▼                                 │
  ⟨      TOMOGRAPHIC DATA UPDATED?             ⟩── NO ─────────┤
                            │                                 │
                           YES                                │
                            ▼                                 │
  S121   ┌──────────────────────────────────────────┐        │
         │         ACQUIRE TOMOGRAPHIC DATA          │        │
         └──────────────────┬───────────────────────┘        │
                            ▼                                 │
  S122   ┌──────────────────────────────────────────┐        │
         │       UPDATE THREE-DIMENSIONAL DATA       │        │
         └──────────────────┬───────────────────────┘        │
                            └────────────────────────────────┘
```

## FIG. 10

# FIG. 11

# FIG. 12

FIG. 13

FIG. 14

# FIG. 15

```
                    ┌─────────────┐
                    │    START    │
                    └─────────────┘
                           │
           ┌───────────────┤
           │               ▼
    ┌──────┴────────────────────────────────────┐
  NO│          FIRST USER OPERATION?             │───S201
    └────────────────────┬──────────────────────┘
                         │ YES
                         ▼
    ┌────────────────────────────────────────────┐
    │       DISPLAY SECOND GRAPHIC ELEMENT        │───S202
    └────────────────────┬───────────────────────┘
                         │
           ┌─────────────┤
           │             ▼
    ┌──────┴────────────────────────────────────┐
  NO│          SECOND USER OPERATION?            │───S203
    └────────────────────┬──────────────────────┘
                         │ YES
                         ▼
    ┌────────────────────────────────────────────┐
    │        DISPLAY THIRD GRAPHIC ELEMENT        │───S204
    └────────────────────┬───────────────────────┘
                         │
                         ▼
    ┌────────────────────────────────────────────┐
    │  CALCULATE INTERMEDIATE POSITION BETWEEN    │
    │ SECOND GRAPHIC ELEMENT AND THIRD GRAPHIC ELEMENT │───S205
    └────────────────────┬───────────────────────┘
                         │
                         ▼
    ┌────────────────────────────────────────────┐
    │        DISPLAY FOURTH GRAPHIC ELEMENT       │───S206
    └────────────────────┬───────────────────────┘
                         │
                         ▼
                  ┌─────────────┐
                  │     END     │
                  └─────────────┘
```

49

## FIG. 16

EP 4 400 059 A1

SETTING OF CUTTING REGION — 81

82 — ☑ ACTIVATE

BASE ANGLE ▬ 83

OPENING ANGLE ▬ 84

USE CENTROID ☑ 85

65 56

71

L1 72

L2 M

87b

87d

87c

87a

86

63 80 53

61

60

54b

54d 66

54c

54a

67 55

64

MOVE TO BOOKMARK

88b
MOVE TO UPPER END

88d
MOVE TO MIDDLE

88c
MOVE TO LOWER END

# FIG. 17

11

IMAGE PROCESSING DEVICE

CONTROL UNIT — 41

51

TOMOGRAPHIC DATA

52

THREE-DIMENSIONAL DATA

53

THREE-DIMENSIONAL IMAGE

MOVEMENT CONTROL FUNCTION ---- 46

42 — STORAGE UNIT

43 — COMMUNICATION UNIT

44 — INPUT UNIT

45 — OUTPUT UNIT

# FIG. 18

EP 4 400 059 A1

# FIG. 19

SETTING OF CUTTING REGION

☑ ACTIVATE
BASE ANGLE
OPENING ANGLE
USE CENTROID ☑

# FIG. 20

# FIG. 21

```
                    ┌──────────────┐
                    │    START     │
                    └──────┬───────┘
                           │
        ┌──────────────────▼──────────────────┐
  NO  ◄─┤       FIRST USER OPERATION?         ├── S211
        └──────────────────┬──────────────────┘
                           │ YES
        ┌──────────────────▼──────────────────┐
        │          ROTATE VIEWPOINT           │── S212
        └──────────────────┬──────────────────┘
                           │
        ┌──────────────────▼──────────────────┐
  NO  ◄─┤       SECOND USER OPERATION?        ├── S213
        └──────────────────┬──────────────────┘
                           │ YES
        ┌──────────────────▼──────────────────┐
        │      REVERSELY ROTATE VIEWPOINT     │── S214
        └──────────────────┬──────────────────┘
                           │
                    ┌──────▼───────┐
                    │     END      │
                    └──────────────┘
```

## INTERNATIONAL SEARCH REPORT

| International application No. |
| --- |
| **PCT/JP2022/034644** |

**A. CLASSIFICATION OF SUBJECT MATTER**

*A61B 8/12*(2006.01)i
FI:  A61B8/12

According to International Patent Classification (IPC) or to both national classification and IPC

**B. FIELDS SEARCHED**

Minimum documentation searched (classification system followed by classification symbols)

A61B8/00-A61B8/15; A61B1/00-A61B1/32; G06T1/00; G06T7/00-G06T7/90

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | US 2020/0129142 A1 (VOLCANO CORPORATION) 30 April 2020 (2020-04-30) | 1-2, 4-5, 14-18 |
| | paragraphs [0078]-[0088], [0091]-[0095], [0106]-[0109], fig. 5-9, 11-13, 21-23 | |
| Y | | 3, 6-13 |
| Y | US 2011/0021903 A1 (MEDIGUIDE LTD) 27 January 2011 (2011-01-27) | 3, 6-13 |
| | paragraphs [0039], [0061]-[0062], fig. 4 | |
| Y | JP 2008-512171 A (MEDIGUIDE LTD) 24 April 2008 (2008-04-24) | 6-13 |
| | paragraphs [0025], [0050]-[0052], fig. 2 | |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 October 2022** | **25 October 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/034644**

| Patent document cited in search report | | | Publication date (day/month/year) | Patent family member(s) | | | Publication date (day/month/year) |
|---|---|---|---|---|---|---|---|
| US | 2020/0129142 | A1 | 30 April 2020 | JP | 2022-509393 | A | |
| | | | | WO | 2020/084039 | A1 | |
| | | | | EP | 3870063 | A1 | |
| | | | | CN | 112912011 | A | |
| US | 2011/0021903 | A1 | 27 January 2011 | US | 2013/0184569 | A1 | |
| | | | | WO | 2008/136008 | A2 | |
| | | | | WO | 2014/137597 | A1 | |
| | | | | EP | 2950709 | A1 | |
| JP | 2008-512171 | A | 24 April 2008 | US | 2005/0107688 | A1 | |
| | | | | paragraphs [0058], [0078]-[0080], fig. 2 | | | |
| | | | | US | 2006/0058647 | A1 | |
| | | | | US | 2010/0331950 | A1 | |
| | | | | US | 2013/0166011 | A1 | |
| | | | | WO | 2006/027781 | A2 | |
| | | | | EP | 1779779 | A2 | |
| | | | | CA | 2616989 | A1 | |
| | | | | IL | 177924 | A | |

Form PCT/ISA/210 (patent family annex) (January 2015)

**EP 4 400 059 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100215238 A
- US 6385332 B
- US 6251072 B